# FASCICULE DE BREVET EUROPEEN

(11) **EP 3 504 001 B1**
(45) Date de publication et mention de la délivrance du brevet: **19.10.2022**
(21) Numéro de dépôt: 17768838.9
(22) Date de dépôt: 28.08.2017
(51) Int. Cl.: B01J 37/26, B01J 38/12, B01J 27/12, B01J 27/128, B01J 27/132, B01J 27/138, B01J 27/32, B01J 35/00, B01J 35/10, C07C 17/20, C07C 21/18

(54) **PROCÉDÉ DE MODIFICATION DE LA DISTRIBUTION EN FLUOR DANS UN COMPOSÉ HYDROCARBURE À L'AIDE D'UNE COMPOSITION DE CATALYSEUR COMPRENANT UN OXYFLUORURE OU FLUORURE DE CHROME**
VERFAHREN ZUR MODIFIZIERUNG DER FLUORVERTEILUNG EINES KOHLENWASSERSTOFFES IN GEGENWART EINES KATALYSATORS ENTHALTEND CHROM-OXYFLUORID ODER -FLUORID
PROCESS OF MODIFICATION OF THE FLUORINE DISTRIBUTION OF A HYDROCARBON WITH A CATALYST COMPOSITION COMPRISING CHROMIUM OXYFLUORIDE OR FLUORIDE

(30) Priorité: 29.08.2016 FR 1657989
(43) Date de publication de la demande: 03.07.2019
(73) Titulaire: Arkema France, 92700 Colombes (FR)
(72) Inventeur: GARRAIT, Dominique, 69390 Charly (FR); PIGAMO, Anne, 69340 Francheville (FR); WENDLINGER, Laurent, 69510 Soucieu En Jarrest (FR)
(74) Mandataire: Arkema Patent
(86) Numéro de dépôt international: PCT/FR2017/052286
(87) Numéro de publication internationale: WO 2018/042114

(56) Documents cités:
- EP-A1- 0 502 605
- EP-A1- 2 223 906
- WO-A1-98/10862
- US-A- 5 741 748
- US-A1- 2013 035 526
- US-A1- 2015 360 218
- Y. ZHU ET AL: "Aliovalent-substituted chromium-based catalysts for the hydrofluorination of tetrachloroethylene", JOURNAL OF CATALYSIS., vol. 219, no. 1, 1 octobre 2003 (2003-10-01), pages 8-16, XP055317026, US ISSN: 0021-9517, DOI: 10.1016/S0021-9517(03)00183-0
- ADAMCZYK B ET AL: "Fluorine modified chromium oxide and its impact on heterogeneously catalyzed fluorination reactions", JOURNAL OF FLUORINE CHEMISTRY, ELSEVIER, NL, vol. 101, no. 2, 1 février 2000 (2000-02-01), pages 239-246, XP004244524, ISSN: 0022-1139, DOI: 10.1016/S0022-1139(99)00165-7
- KATHARINA TEINZ ET AL: "Catalytic formation of 2,3,3,3-tetrafluoropropene from 2-chloro-3,3,3-trifluoropropene at fluorinated chromia: A study of reaction pathways", APPLIED CATALYSIS. B, ENVIRONMENTAL, vol. 165, 1 avril 2015 (2015-04-01), pages 200-208, XP055212251, ISSN: 0926-3373, DOI: 10.1016/j.apcatb.2014.09.076
- David W. Bonniface ET AL: "Halogen exchange reactions for CFC alternatives.. The behaviour of fluorine-18 labelled hydrogen fluoride towards prefluorinated chromia containing nickel(II) or zinc(II)", Green Chemistry, 1999 (1), 1 février 1999 (1999-02-01), pages 9-11, XP055366766, Extrait de l'Internet: URL:http://pubs.rsc.org/en/content/article pdf/1999/gc/a808021f [extrait le 2017-04-24]
- WEI MAO ET AL: "Catalytic gas-phase fluorination of 1,1,2,3-tetrachloropropene to 2-chloro-3,3,3-trifluoropropene over the fluorinated Cr2O3-based catalysts", APPLIED CATALYSIS A: GENERAL, vol. 491, 1 février 2015 (2015-02-01), pages 37-44, XP055214016, ISSN: 0926-860X, DOI: 10.1016/j.apcata.2014.11.027

## Description

### Domaine technique

La présente invention se rapporte à des compositions catalytiques d'oxyfluorure ou de fluorure de chrome et leur utilisation dans un procédé en phase gazeuse. La présente invention concerne aussi la préparation desdites compositions catalytiques d'oxyfluorure ou de fluorure de chrome.

### Contexte de l'invention

Les hydrocarbures halogénés, en particulier les hydrocarbures fluorés comme les hydrofluorooléfines, sont des composés qui ont une structure utile comme matériaux fonctionnels, solvants, réfrigérants, agents de soufflage et monomères pour polymères fonctionnels ou matériaux de départ pour de tels monomères. Des hydrofluorooléfines comme le 2,3,3,3-tétrafluoropropène (HFO-1234yf) attirent l'attention parce qu'elles offrent un comportement prometteur comme réfrigérants à faible potentiel de réchauffement global.

Les procédés de production de fluorooléfines sont habituellement effectués en présence d'une substance de départ telle qu'un alcane contenant du chlore ou un alcène contenant du chlore, et en présence d'un agent fluorant tel que le fluorure d'hydrogène. Ces procédés peuvent être effectués en phase gazeuse ou en phase liquide, en absence ou non de catalyseur.

Les procédés en phase gazeuse sont habituellement effectués en présence de catalyseurs, en particulier en présence de catalyseurs à base de chrome. US 2015/0148571 dévoile un procédé de production d'oléfine contenant du fluor où le catalyseur est de l'oxyde de chrome fortement cristallisé. WO 2005/037431 dévoile une composition catalytique contenant du chrome comprenant ZnCr₂O₄ et un oxyde de chrome α cristallin et son utilisation dans un procédé pour modifier la distribution de fluor dans un hydrocarbure halogéné ou pour incorporer du fluor dans un hydrocarbure saturé ou insaturé. WO 2007/019353 dévoile la fabrication de 1,1,1,3,3-pentafluoropropane et 1,1,1,2,3-pentafluoropropane à partir d'un halopropène de formule CX₃CCI=CCIX en présence d'un oxyde de chrome α cristallin, où au moins 0,05 % des atomes de chrome dans le réseau de l'oxyde de chrome α sont remplacés par un cuivre divalent. WO 98/10862 dévoile un catalyseur de fluoration à base d'oxyde de chrome III, dans lequel l'oxyde de chrome III est au moins partiellement et peut contenir un atome de zinc ou un de ses composés. Le catalyseur a été utilisé dans un procédé de fabrication de HFC-134a. Un oxyde fluorochrome ayant une teneur en fluor d'au moins 30 % en poids est également utilisé comme catalyseur dans un procédé de production d'oléfines contenant du fluor comme dévoilé dans EP 2 223 906. Zhu et al., Journal of Catalysis 219 (2003) 8-16, décrivent des catalyseurs d'oxyde de chrome comprenant du Zr ou du V pour l'hydrofluoration du tétrachloroéthylène. Adamczyk et al., Journal of Fluorine Chemistry 101 (2000) 239-246, décrivent des catalyseurs d'oxyfluorure de chrome. Teinz et al., Applied Catalysis B : Environmental 165 (2015) 200-208, décrivent la formation catlaytique du HFO-1234yf à partir de HCFO-1233xf en présence d'un catalyseur F-Cr₂O₃. Bonniface et al., Green Chemistry, February 1999, 9-11, décrivent des réactions d'échange d'halogène. US 5,741,748 décrit un procédé pour éliminer le fluor d'un catalyseur de chrome usagé. Mao et al., Applied Catalysis A : General 491 (2015) 37-44 décrit la fluoration en phase gazeuse de 1,1,2,3-tétrachloropropène en 2-chloro-3,3,3-trifluoropropène en présence d'un catalyseur basé sur Cr₂O₃. US 2015/0360218 décrit un procédé d'activation d'un catalyseur de fluoration en présence de NF₃ ou F₂. EP 0502605 décrit des catalyseurs de chrome comprenant du zinc. US 2013/0035526 décrit un procédé de production du HFO-1234yf à partir du HCFC-241bb.

Il existe également un besoin pour des compositions catalytiques ayant une forte activité (conversion) et/ou sélectivité ainsi que pour des procédés chimiques industriels sur la durée de vie d'un catalyseur.

### Résumé de l'invention

Dans un premier aspect, la présente invention se rapporte à un procédé de modification de la distribution en fluor dans un composé hydrocarbure de formule (I) CX(Y)₂-CX(Y)ₘ-CHₘXY où X et Y représentent indépendamment H, F ou Cl et m = 0 ou 1 avec au moins un parmi X ou Y qui est Cl ou F ; en présence d'un catalyseur, le catalyseur étant une composition solide contenant au moins un composant contenant de l'oxyfluorure ou du fluorure de chrome de formule brute CrₓM(₁₋ₓ)OᵣFₛ, où 2r+s est supérieur ou égal à 2,9 et inférieur à 6, M est Zn, x vaut de 0,9 à 0,999, s est supérieur à 0 et inférieur ou égal à 6 et r est supérieur ou égal à 0 et inférieur à 3, caractérisé en ce que ledit composant contenant de l'oxyfluorure ou du fluorure de chrome est amorphe, et en ce que la composition solide a une surface spécifique entre 15 et 45 m²/g

Dans une réalisation préférentielle, ledit composant contenant de l'oxyfluorure ou du fluorure de chrome de formule brute CrₓM(₁₋ₓ)OᵣFₛ où M est Zn; en particulier x vaut 0,94 à 0,99.

Dans une réalisation préférentielle, ledit composé hydrocarbure est choisi dans le groupe constitué de tétrachloropropène, chlorotrifluoropropène, pentachloropropane, dichlorotrifluoropropane, trichlorodifluoropropane, tétrachlorofluoropropane, dichlorodifluoropropène, trichlorofluoropropène, tétrafluorochloropropane, pentafluoropropane et leur mélanges ; de préférence, le composé hydrocarbure est choisi dans le groupe constitué de 2-chloro-3,3,3-trifluoro-1-propène (HFCO-1233xf), 2,3-dichloro-1,1,1-trifluoropropane (HCFC-243db), 1,1,1,2,3-pentachloropropane (HCC-240db), 1,1,2,2,3-pentachloropropane (HCC-240aa), 1,1,1,3,3-pentachloropropane (HCC-240fa), 1,1,2,3-tétrachloro-1-propène (HCO-1230xa), 2,3,3,3-tétrachloro-1-propène (HCO-1230xf), 1,1,3,3-tétrachloro-1-propène (HCO-1230za), 1,3,3,3-tétrachloro-1-propène (HCO-1230zd), 1,1,1,2,2-pentafluoropropane (HFC-245cb) et 1-chloro-3,3,3-trifluoro-1-propène (HCFO-1233zd).

Dans une réalisation préférentielle, on augmente la teneur en fluor du composé hydrocarbure en faisant réagir ledit composé avec du fluorure d'hydrogène en phase gazeuse en présence de ladite composition solide, le composé hydrocarbure étant un hydrocarbure halogéné saturé ou un hydrocarbure halogéné insaturé ou un hydrocarbure insaturé.

Dans une réalisation préférentielle, on diminue la teneur en fluor du composé hydrocarbure par déshydrofluoration, ledit composé hydrocarbure étant en présence de ladite composition solide, ledit composé hydrocarbure étant un composé hydrocarbure fluoré.

Sinon, on diminue la teneur en chlore du composé hydrocarbure par déshydrochloration, ledit composé hydrocarbure étant en présence de ladite composition solide, ledit composé hydrocarbure étant un composé hydrocarbure chloré.

Dans une réalisation préférentielle, on modifie la distribution en fluor dans le composé hydrocarbure en isomérisant ledit composé hydrocarbure en présence de ladite composition solide, ledit composé hydrocarbure étant un composé hydrocarbure fluoré.

Dans une réalisation préférentielle, on modifie la distribution en fluor dans le composé hydrocarbure en dismutant ledit composé hydrocarbure dans la phase gazeuse en présence de ladite composition solide, ledit composé hydrocarbure étant un composé hydrocarbure chlorofluoré.

Dans une réalisation préférentielle, on diminue la teneur en fluor du composé hydrocarbure en faisant réagir ledit composé hydrocarbure avec du chlorure d'hydrogène dans la phase gazeuse en présence de ladite composition solide, ledit composé hydrocarbure étant un composé hydrocarbure halogéné comprenant au moins un atome de fluor.

Dans une réalisation préférentielle, on augmente la teneur en fluor d'un premier composé hydrocarbure en faisant réagir ledit premier composé hydrocarbure avec du fluorure d'hydrogène en phase gazeuse en présence d'une composition solide, le premier composé hydrocarbure étant un hydrocarbure halogéné saturé ou un hydrocarbure halogéné insaturé ou un hydrocarbure insaturé, et on diminue la teneur en fluor d'un deuxième composé hydrocarbure par déshydrofluoration dudit deuxième composé hydrocarbure en présence de ladite composition solide, ledit deuxième composé hydrocarbure étant un composé hydrocarbure fluoré.

Dans une réalisation préférentielle, on augmente la teneur en fluor d'un premier composé hydrocarbure en faisant réagir ledit composé hydrocarbure avec du fluorure d'hydrogène en phase gazeuse en présence d'une composition solide, le premier composé hydrocarbure étant un hydrocarbure halogéné saturé ou un hydrocarbure halogéné insaturé ou un hydrocarbure insaturé, et on diminue la teneur en fluor d'un deuxième composé hydrocarbure en déshydrochlorant ledit deuxième composé hydrocarbure en présence de ladite composition solide, ledit deuxième composé hydrocarbure étant un composé hydrocarbure fluoré.

Dans une réalisation préférentielle, le composant contenant de l'oxyfluorure ou du fluorure de chrome est de formule brute CrₓM(₁₋ₓ)OᵣFₛ, où r est supérieur ou égal à 0 et inférieur à 2,62, s est supérieur ou égal à [(0,16 r) + 0,52)]/0,92 à condition que s soit supérieur ou égal à 0,76 et inférieur à 6.

Dans une réalisation préférentielle, la composition solide comprend un support choisi dans le groupe constitué du charbon actif, de l'alumine, du fluorure de magnésium et du fluorure d'aluminium.

Dans une réalisation préférentielle, le composant contenant de l'oxyfluorure ou du fluorure de chrome est de formule brute CrₓM(₁₋ₓ)OᵣFₛ, où r est supérieur ou égal à 0 et inférieur à 2,05, s est supérieur ou égal à [(0,4 r) + 1,3)]/0,8 à condition que s soit supérieur ou égal à 1,90 et inférieur à 6, de préférence de formule brute CrₓM(₁₋ₓ)OᵣFₛ où r est supérieur ou égal à 0 et inférieur à 1,75, s est supérieur ou égal à [(0,56 r) + 1,82)]/0,72 à condition que s soit supérieur ou égal à 2,50 et inférieur à 6, et une surface spécifique entre 15 et 45 m²/g.

Dans une réalisation préférentielle, le composant contenant de l'oxyfluorure ou du fluorure de chrome est de formule brute CrOᵣFₛ, où r est supérieur ou égal à 0 et inférieur à 2,05, s est supérieur ou égal à [(0,4 r) + 1,3)]/0,8 à condition que s soit supérieur ou égal à 1,90 et inférieur à 6, de préférence de formule brute CrₓM(₁₋ₓ)OᵣFₛ où r est supérieur ou égal à 0 et inférieur à 1,75, s est supérieur ou égal à [(0,56 r) + 1,82)]/0,72 à condition que s soit supérieur ou égal à 2,50 et inférieur à 6, et une surface spécifique entre 15 et 45 m²/g.

### Brève description des illustrations

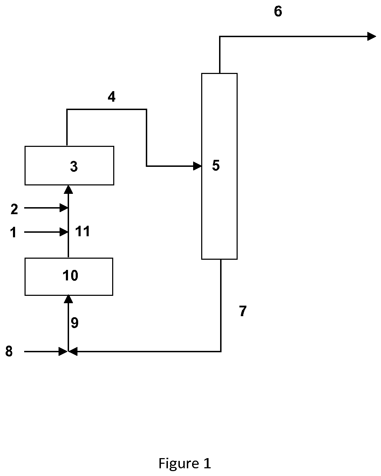

La Figure 1 représente une vue schématique d'un procédé selon une réalisation de la présente invention.

### Description détaillée de l'invention

Dans la présente invention, il est fourni un procédé de modification de la distribution en fluor dans un composé hydrocarbure en présence d'un catalyseur. Dans ce procédé, une composition solide comprenant au moins un composant contenant de l'oxyfluorure ou du fluorure de chrome de formule brute CrₓM(₁₋ₓ)OᵣFₛ, où 2r+s est supérieur ou égal à 2,9 et inférieur à 6, M est Zn, x vaut entre 0,9 et 0,999, s est supérieur à 0 et inférieur ou égal à 6 et r est supérieur ou égal à 0 et inférieur à 3, ledit composant contenant de l'oxyfluorure ou du fluorure de chrome étant amorphe, la composition solide ayant une surface spécifique entre 15 et 45 m2/g, est utilisée comme catalyseur.

On peut utiliser ledit catalyseur pour modifier la distribution en fluor dans des composés hydrocarbures, ces derniers étant des composés hydrocarbures halogénés ou non. On peut modifier la distribution en fluor dans un composé hydrocarbure en augmentant la teneur en fluor du composé hydrocarbure. On peut aussi modifier la distribution en fluor d'un composé hydrocarbure en diminuant la teneur en fluor du composé hydrocarbure et/ou en réarrangeant la position d'atomes de fluor sur les atomes de carbone du composé hydrocarbure.

La présente invention fournit des procédés où on modifie la distribution en fluor dans des composés hydrocarbures halogénés contenant trois atomes de carbone. La présente invention peut fournir des procédés où on augmente la teneur en fluor de composés hydrocarbures halogénés contenant trois atomes de carbone. Les procédés de modification de la distribution en fluor de composés hydrocarbures, de préférence de composés hydrocarbures halogénés, incluent la fluoration, la chlorofluoration, l'isomérisation, dismutation, déshydrofluoration et chlorodéfluoration.

En particulier, ledit composant contenant de l'oxyfluorure ou du fluorure de chrome est amorphe. Le terme amorphe se réfère à un solide dans lequel les atomes n'ont pas une structure atomique ordonnée. Les solides amorphes n'ont pas de structures internes répétitives à longue distance, comme on en trouve dans les cristaux. Le composant contenant de l'oxyfluorure ou du fluorure de chrome est amorphe quand on ne détecte aucun signal par cristallographie aux rayons X représentatif de formes cristallines (figure de diffraction cristalline).

Le composant contenant de l'oxyfluorure ou du fluorure de chrome est de formule brute CrₓM(₁₋ₓ)OᵣFₛ, où M est du Zn. La composition solide contient du zinc à hauteur d'entre 0,1 et 10 % en moles rapporté à la composition solide, en particulier à hauteur d'entre 1 et 6 % en moles rapporté à la composition solide. Par conséquent, le composant contenant de l'oxyfluorure ou du fluorure de chrome **est** de formule brute CrₓZn(₁₋ₓ)OᵣFₛ, où x vaut entre 0,9 et 0,999, plus préférentiellement entre 0,94 et 0,99.

Les composés hydrocarbures qui conviennent aux procédés selon la présente invention sont de formule (I) CX(Y)₂-CX(Y)ₘ-CHₘXY, où X et Y représentent indépendamment H, F ou Cl et m = 0 ou 1 avec au moins un parmi X ou Y étant Cl ou F. De préférence, les composés hydrocarbures peuvent être choisis dans le groupe constitué de tétrachloropropène, chlorotrifluoropropène, pentachloropropane, dichlorotrifluoropropane, trichlorodifluoropropane, tétrachlorofluoropropane, dichlorodifluoropropène, trichlorofluoropropène, pentafluoropropane et leurs mélanges.

De préférence, les composés hydrocarbures peuvent être choisis dans le groupe constitué de 2-chloro-3,3,3-trifluoro-1-propène (HFCO-1233xf), 2,3-dichloro-1,1,1-trifluoropropane (HCFC-243db), 1,1,1,2,3-pentachloropropane (HCC-240db), 1,1,2,2,3-pentachloropropane (HCC-240aa), 1,1,1,3,3-pentachloropropane (HCC-240fa), 1,1,2,3-tétrachloro-1-propène (HCO-1230xa), 2,3,3,3-tétrachloro-1-propène (HCO-1230xf), 1,1,3,3-tétrachloro-1-propène (HCO-1230za), 1,3,3,3-tétrachloro-1-propène (HCO-1230zd), 1,1,1,2,2-pentafluoropropane (HFC-245cb) et 1-chloro-3,3,3-trifluoro-1-propène (HCFO-1233zd).

De préférence, le composant contenant de l'oxyfluorure ou du fluorure de chrome peut être de formule brute CrₓM₍₁₋ₓ₎OᵣFₛ, où r est supérieur ou égal à 0 et inférieur à 2,62, s est supérieur ou égal à [(0,16 r) + 0,52)]/0,92 à condition que s soit supérieur ou égal à 0,76 et inférieur à 6, plus préférentiellement le composant contenant de l'oxyfluorure ou du fluorure de chrome est de formule brute CrₓM₍₁₋ₓ₎OᵣFₛ, où r est supérieur ou égal à 0 et inférieur à 2,43, s est supérieur ou égal à [(0,24 r) + 0,78)]/0,88 à condition que s soit supérieur ou égal à 1,14 et inférieur à 6, plus préférentiellement le composant contenant de l'oxyfluorure ou du fluorure de chrome est de formule brute CrₓM₍₁₋ₓ₎OᵣFₛ, où r est supérieur ou égal à 0 et inférieur à 2,24, s est supérieur ou égal à [(0,32 r) + 1,04)]/0,84 à condition que s soit supérieur ou égal à 1,52 et inférieur à 6, encore plus préférentiellement le composant contenant de l'oxyfluorure ou du fluorure de chrome est de formule brute CrₓM₍₁₋ₓ₎OᵣFₛ, où r est supérieur ou égal à 0 et inférieur à 2,05, s est supérieur ou égal à [(0,4 r) + 1,3)]/0,8 à condition que s soit supérieur ou égal à 1,90 et inférieur à 6, en particulier le composant contenant de l'oxyfluorure ou du fluorure de chrome est de formule brute CrₓM₍₁₋ₓ₎OᵣFₛ, où r est supérieur ou égal à 0 inférieur à 1,75, s est supérieur ou égal à [(0,56 r) + 1,82)]/0,72 à condition que s soit supérieur ou égal à 2,50 et inférieur à 6. Plus particulièrement, le composé contenant de l'oxyfluorure ou du fluorure de chrome est de formule brute CrₓM₍₁₋ₓ₎OᵣFₛ comme défini dans la présente et est amorphe, et plus préférentiellement anhydre et amorphe. Le composant contenant du fluorure de chrome peut être CrₓM₍₁₋ₓ₎F₃, amorphe, plus préférentiellement un CrₓM₍₁₋ₓ₎F₃ amorphe anhydre.

La composition solide peut comprendre un support choisi dans le groupe constitué de charbon actif, alumine, graphite, fluorure de magnésium et fluorure d'aluminium. La composition solide a une surface spécifique entre 15 et 45 m²/g.

Un catalyseur encore plus préférentiel est une composition solide comprenant un composant contenant de l'oxyfluorure ou du fluorure de chrome de formule brute CrₓM₍₁₋ₓ₎OᵣFₛ, où r est supérieur ou égal à 0 et inférieur à 2,05, s est supérieur ou égal à [(0,4 r) + 1,3)]/0,8 à condition que s soit supérieur ou égal à 1,90 et inférieur à 6, de préférence de formule brute CrₓM₍₁₋ₓ₎OᵣFₛ, où r est supérieur ou égal à 0 et inférieur à 1,75, s est supérieur ou égal à [(0,56 r) + 1,82)]/0,72 à condition que s soit supérieur ou égal à 2,50 et inférieur à 6, et une surface spécifique entre 15 et 45 m²/g. En particulier, la composition solide comprend un composant contenant de l'oxyfluorure ou du fluorure de chrome de formule brute CrₓM₍₁₋ₓ₎F₃ et ayant une surface spécifique entre 15 et 45 m²/g. Le composant contenant de l'oxyfluorure ou du fluorure de chrome est amorphe. M est du zinc.

Dans une première réalisation, on augmente la teneur en fluor du composé hydrocarbure en faisant réagir ledit composé avec du fluorure d'hydrogène en présence de ladite composition solide, le composé hydrocarbure étant un hydrocarbure halogéné saturé ou un hydrocarbure halogéné insaturé ou un hydrocarbure insaturé.

Des composés hydrocarbures adéquats comme réactifs de départ pour le procédé de fluoration de cette première réalisation peuvent être des composés hydrocarbures halogénés saturés ou insaturés.

On peut augmenter la teneur en fluor des composés hydrocarbures saturés halogénés répondant à la formule (I)définie dans la revendication 1 en faisant réagir lesdits composés hydrocarbures avec HF en phase vapeur en présence d'un catalyseur étant une composition solide comprenant au moins un composant contenant de l'oxyfluorure ou du fluorure de chrome de formule brute CrₓM(₁₋ₓ)OᵣFₛ, où 2r+s est supérieur ou égal à 2,9 et inférieur à 6, M est Zn, x vaut entre 0,9 et 0,999, s est supérieur à 0 et inférieur ou égal à 6 and r est supérieur ou égal à 0 et inférieur à 3, ladite composition solide est amorphe.

Préférentiellement, le composant contenant de l'oxyfluorure ou du fluorure de chrome peut être de formule brute CrₓM(₁₋ₓ)OᵣFₛ, où r est supérieur ou égal à 0 et inférieur à 2,62, s est supérieur ou égal à [(0,16 r) + 0,52)]/0,92 à condition que s soit supérieur ou égal à 0,76 et inférieur à 6, plus préférentiellement le composant contenant de l'oxyfluorure ou du fluorure de chrome est de formule brute CrₓM(₁₋ₓ)OᵣFₛ, où r est supérieur ou égal à 0 et inférieur à 2,43, s est supérieur ou égal à [(0,24 r) + 0,78)]/0,88 à condition que s soit supérieur ou égal à 1,14 et inférieur à 6, idéalement le composant contenant de l'oxyfluorure ou du fluorure de chrome est de formule brute CrₓM(₁₋ₓ)OᵣFₛ, où r est supérieur ou égal à 0 et inférieur à 2,24, s est supérieur ou égal à [(0,32 r) + 1,04)]/0,84 à condition que s soit supérieur ou égal à 1,52 et inférieur à 6, encore plus préférentiellement le composé contenant de l'oxyfluorure ou du fluorure de chrome est de formule brute CrₓM(₁₋ₓ)OᵣFₛ, où r est supérieur ou égal à 0 et inférieur à 2,05, s est supérieur ou égal à [(0,4 r) + 1,3)]/0,8 à condition que s soit supérieur ou égal à 1,90 et inférieur à 6, en particulier le composant contenant de l'oxyfluorure ou du fluorure de chrome est de formule brute CrₓM(₁₋ₓ)OᵣFₛ, où r est supérieur ou égal à 0 et inférieur à 1,75, s est supérieur ou égal à [(0,56 r) + 1,82)]/0,72 à condition que s soit supérieur ou égal à 2,50 et inférieur à 6. En particulier M est du zinc. Par conséquent, le composant contenant de l'oxyfluorure ou du fluorure de chrome est de formule brute CrₓZn(₁₋ₓ)OᵣFₛ, où x vaut entre 0,9 et 0,999, plus préférentiellement entre 0,94 et 0,99 ; avec r et s comme définis plus haut.

Dans une réalisation préférentielle, la composition solide comprend un support choisi dans le groupe constitué de charbon actif, alumine et fluorure d'aluminium. La composition solide a une surface spécifique entre 15 et 45 m²/g.

En particulier, le composant contenant de l'oxyfluorure ou du fluorure de chrome est de formule brute CrₓM(₁₋ₓ)OᵣFₛ, où r est supérieur ou égal à 0 et inférieur à 2,05, s est supérieur ou égal à [(0,4 r) + 1,3)]/0,8 à condition que s soit supérieur ou égal à 1,90 et inférieur à 6, de préférence de formule brute CrₓM(₁₋ₓ)OᵣFₛ, où r est supérieur ou égal à 0 et inférieur à 1,75, s est supérieur ou égal à [(0,56 r) + 1,82)]/0.72 à condition que s soit supérieur ou égal à 2,50 et inférieur à 6, et une surface spécifique entre 15 et 45 m²/g. M est du zinc. Par conséquent, le composant contenant de l'oxyfluorure ou du fluorure de chrome peut être de formule brute CrₓZn(₁₋ₓ)OᵣFₛ, où x vaut entre 0,9 et 0,999, plus préférentiellement entre 0,94 et 0,99 ; avec r et s comme définis plus haut et avec une surface spécifique entre 15 et 45 m²/g.

Le procédé selon la première réalisation peut être mené dans un réacteur comprenant un lit catalytique contenant un catalyseur et suivant les conditions opératoires suivantes :
- un rapport molaire HF/composé hydrocarbure entre 1:1 et 150:1, de préférence entre 2:1 et 125:1, plus préférentiellement entre 3:1 et 100:1 ;
- un temps de contact entre 1 et 100 s, de préférence entre 2 et 75 s, en particulier entre 3 et 50 s;
- une pression entre la pression atmosphérique et 20 bar, de préférence entre 2 et 18 bar, plus préférentiellement entre 3 et 15 bar ;
- une température (du lit catalytique) entre 200 et 450°C, de préférence entre 250 et 400°C, plus préférentiellement entre 280°C et 380°C.

Le procédé peut être mené à bien sur une durée comprise entre 10 et 8000 h, de préférence entre 50 et 5000 h, plus préférentiellement entre 70 et 1000 h.

On peut ajouter un oxydant, comme l'oxygène ou le chlore, en cours de procédé. Le rapport molaire de l'oxydant sur le composé hydrocarbure peut être entre 0,005 et 2, de préférence entre 0,01 et 1,5. L'oxydant peut être de l'oxygène pur, de l'air ou un mélange d'oxygène et d'azote.

Le procédé est mené typiquement dans un réacteur tubulaire. Le réacteur et ses lignes d'alimentation associées, lignes d'effluent et appareils associés doivent être construits dans des matériaux résistants au fluorure d'hydrogène et au chlorure d'hydrogène. Les matériaux de construction typiques, bien connus en l'état de l'art de la fluoration, incluent les aciers inox, en particulier de type austénitique, les alliages bien connus à haute teneur en nickel, comme les alliages nickel-cuivre Monel^{®}, les alliages à base de nickel Hastelloy^{®} et les alliages nickel-chrome Inconel^{®}.

La quantité de HF mis à réagir avec les composés hydrocarbures doit être au moins stœchiométrique. La quantité stœchiométrique est basée sur le nombre de substituants H, Br et/ou Cl à remplacer par F en plus d'une mole de HF pour saturer la double liaison carbonecarbone s'il y en a.

Des exemples de composés halogénés saturés de formule CₙHₐBr_{b}Cl_{c}F_{d} qui peuvent être mis à réagir avec HF en présence des catalyseurs de cette invention incluent C₃HCl₇, C₃HCl₆F, C₃HCl₅F₂, C₃HCl₄F₃, C₃HCl₃F₄, C₃HCl₂F₅, C₃H₂Cl₆, C₃H₂BrCl₅, C₃H₂Cl₅F, C₃H₂Cl₄F₂, C₃H₂Cl₃F₃, C₃H₂Cl₂F₄, C₃H₂ClF₅, C₃H₃Cl₅, C₃H₃Cl₄F, C₃H₃Cl₃F₂, C₃H₃Cl₂F₃, C₃H₃ClF₄, C₃H₄Cl₄, répondant à la formule brute (I) définie à revendication 1.

Des exemples spécifiques de réactions de fluoration de composés hydrocarbures saturés halogénés qui peuvent être menées à bien dans les conditions décrites ci-dessus en utilisant les catalyseurs de cette invention incluent la conversion du 1,1,1,3,3-pentachloropropane (CCl₃CH₂CHCl₂ ou HCC-240fa) en un mélange de 1,1,1,3,3-pentafluoropropane (CHF₂CH₂CF₃ ou HFC-245fa), 1-chloro-3,3,3-trifluoro-1-propène (CHCl=CHCF₃ ou HCFO-1233zd) et 1,3,3,3-tétrafluoropropène (CHF=CHCF₃ ou HFO-1234ze), la conversion d'un mélange contenant du 1,1-dichloro-2,2,3,3,3-pentafluoropropane (CF₃CF₂CHCl₂ ou HCFC-225ca) et 1,3-dichloro-1,2,2,3,3-pentafluoropropane (CCIF₂CF₂CHCIF ou HCFC-225cb) en un mélange de 1-chloro-1,2,2,3,3,3-hexafluoropropane (CF₃CF₂CHClF ou HCFC-226ca) et 1,1,1,2,2,3,3-heptafluoropropane (CF₃CF₂CHF₂ ou HFC-227ca), la conversion du 1,1,1,2,3-pentachloropropane (CCl₃CHClCH₂Cl ou HCC-240db) en 2-chloro-3,3,3-trifluoro-1-propène (CF₃CCl=CH₂ ou HCFO-1233xf), la conversion du 1,1,2,2,3-pentachloropropane (CHCl2CCl2CH₂Cl ou HCC-240aa) en 2-chloro-3,3,3-trifluoro-1-propène (CF₃CCl=CH₂ ou HCFO-1233xf), la conversion du 1,1,1,2,3-pentachloropropane (CCl₃CHClCH₂Cl ou HCC-240db) en 2,3,3,3-tétrafluoropropène (CF₃CF=CH₂ ou HFO-1234yf), la conversion du 1,1,2,2,3-pentachloropropane (CHCl₂CCl₂CH₂Cl ou HCC-240aa) en 2,3,3,3-tétrafluoropropène (CF₃CF=CH₂ ou HFO-1234yf), la conversion du 1,1,1,3,3-pentachloropropane (CCl₃CH₂CHCl₂ ou HCC-240fa) en 1,3,3,3-tétrafluoropropène (CF₃CH=CHF ou HFO-1234ze), en particulier la conversion du 1,1,1,2,3-pentachloropropane (CCl₃CHClCH₂Cl ou HCC-240db) en 2-chloro-3,3,3-trifluoro-1-propène (CF₃CCl=CH₂ ou HCFO-1233xf), la conversion du 1,1,2,2,3-pentachloropropane (CHCl₂CCl₂CH₂Cl ou HCC-240aa) en 2-chloro-3,3,3-trifluoro-1-propène (CF₃CCl=CH₂ ou HCFO-1233xf), la conversion du 1,1,1,2,3-pentachloropropane (CCl₃CHClCH₂Cl ou HCC-240db) en 2,3,3,3-tétrafluoropropène (CF₃CF=CH₂ ou HFO-1234yf), la conversion du 1,1,2,2,3-pentachloropropane (CHCl₂CCl₂CH₂Cl ou HCC-240aa) en 2,3,3,3-tétrafluoropropène (CF₃CF=CH₂ ou HFO-1234yf), la conversion du 1,1,1,3,3-pentachloropropane (CCl₃CH₂CHCl₂ ou HCC-240fa) en 1,3,3,3-tétrafluoropropène (CF₃CH=CHF ou HFO-1234ze).

Des exemples de composés insaturés halogénés de formule CₚHₑBr_{f}Cl_{g}Fₕ et CᵢHᵢ qui peuvent être mis à réagir avec HF en présence des catalyseurs de cette invention C₃H₅Cl, C₃H₄Cl₂, C₃H₃Cl₃, C₃H₂Cl₄, C₃HCl₅, C₃H₂CIF₃, C₃F₃HCl₂, C₃F₂H₂Cl₂, C₃F₄H, ClC₃Cl₆, C₃Cl₅F, C₃Cl₄F₂, C₃Cl₃F₃, C₃Cl₂F₄, C₃ClF₅, C₃HF₅, C₃H₂F₄, C₃F₆, répondant à la formule (I) définie à la revendication 1.

Des exemples spécifiques de réactions de fluoration de composés hydrocarbures insaturés halogénés qui peuvent être menées à bien en utilisant les catalyseurs de cette invention incluent la conversion du 1,1,2-trichloro-3,3,3-trifluoro-1-propène (CCl₂=CClCF₃ ou CFC-1213xa) en un mélange de 2,3-dichloro-1,1,1,3,3-pentafluoropropane (CF₃CHClCClF₂ ou HCFC-225da), de 2-chloro-1,1,1,3,3,3-hexafluoropropane (CF₃CHClCF₃ ou HCFC-226da) et/ou de 2-chloro-1,1,3,3,3-pentafluoro-1-propène (CF₃CCl=CF₂ ou CFC-1215xc), la conversion de l'hexafluoropropène (CF₃CF=CF₂ ou CFC-1216yc) en 1,1,1,2,3,3,3-heptafluoropropane (CF₃CHFCF₃ ou HFC-227ea), la conversion du 1,1,3,3,3-pentafluoropropène (CF₃CH=CF₂ ou HFO-1225zc) en 1,1,1,3,3,3-hexafluoropropane (CF₃CH₂CF₃ ou HFC-236fa), la conversion du 1,3,3,3-tétrafluoropropène (CF₃CH=CHF ou HFO-1234ze) en 1,1,1,3,3-pentafluoropropane (CF₃CH₂CHF₂ ou HFC-245fa), la conversion du 2-chloro-3,3,3-trifluoro-1-propène (CF₃CCl=CH₂ ou HCFO-1233xf) en 2,3,3,3-tétrafluoropropène (CF₃CF=CH₂ ou HFO-1234yf), la conversion du 1,1,2,3-tétrachloro-1-propène (CCb=CClCH₂Cl ou HCO-1230xa) en 2-chloro-3,3,3-trifluoro-1-propène (CF₃CCl=CH₂ ou HCFO-1233xf) ou 2,3,3,3-tétrafluoropropène (CF₃CF=CH₂ ou HFO-1234yf), la conversion du 2,3,3,3-tétrachloro-1-propène (CCl₃CCl=CH₂ ou HCO-1230xf) en 2-chloro-3,3,3-trifluoro-1-propène (CF₃CCl=CH₂ ou HCFO-1233xf) ou en 2,3,3,3-tétrafluoropropène (CF₃CF=CH₂ ou HFO-1234yf), la conversion du 1-chloro-3,3,3-trifluoro-1-propène (CF₃CH=CHCl ou HCFO-1233zd) ou du 1,1,3,3-tétrachloro-1-propène (CCl₂=CHCHCl₂ ou HCO-1230za) ou du 1,3,3,3-tétrachloroprop-1-ène (CCl₃CH=CHCl ou HCO-1230zd) en 1,3,3,3-tétrafluoropropène (CF₃CH=CHF ou HFO-1234ze), en particulier la conversion du 2-chloro-3,3,3-trifluoro-1-propène (CF₃CCl=CH₂ ou HCFO-1233xf) en 2,3,3,3-tétrafluoropropène (CF₃CF=CH₂ ou HFO-1234yf), la conversion du 1,1,2,3-tétrachloro-1-propène (CCl₂=CClCH₂Cl ou HCO-1230xa) en 2-chloro-3,3,3-trifluoro-1-propène (CF₃CCl=CH₂ ou HCFO-1233xf) ou en 2,3,3,3-tétrafluoropropène (CF₃CF=CH₂ ou HFO-1234yf), la conversion du 2,3,3,3-tétrachloro-1-propène (CCl₃Cl=CH₂ ou HCO-1230xf) en 2-chloro-3,3,3-trifluoro-1-propène (CF₃CCl=CH₂ ou HCFO-1233xf) ou en 2,3,3,3-tétrafluoropropène (CF₃CF=CH₂ ou HFO-1234yf), la conversion du 1-chloro-3,3,3-trifluoro-1-propène (CF₃CH=CHCl ou HCFO-1233zd) ou du 1,1,3,3-tétrachloro-1-propène (CCl₂=CHCHCl₂ ou HCO-1230za) ou du 1,3,3,3-tétrachloroprop-1-ène (CCl₃CH=CHCl ou HCO-1230zd) en 1,3,3,3-tétrafluoropropène (CF₃CH=CHF ou HFO-1234ze).

Préférentiellement, le composé hydrocarbure est choisi dans le groupe constitué du 2-chloro-3,3,3-trifluoro-1-propène (HFCO-1233xf), 1,1,1,2,3-pentachloropropane (HCC-240db), 1,1,2,2,3-pentachloropropane (HCC-240aa), 1,1,2,3-tétrachloro-1-propène (HCO-1230xa), 2,3,3,3-tétrachloro-1-propène (HCO-1230xf), ou leurs mélanges, pour la production du 2,3,3,3-tétrafluoropropène (HFO-1234yf).

Sinon, le composé hydrocarbure est choisi dans le groupe constitué de 1-chloro-3,3,3-trifluoro-1-propène (HCFO-1233zd), 1,1,1,3,3-pentachloropropane (HCC-240fa), 1,1,3,3-tétrachloro-1-propène (HCO-1230za), 1,3,3,3-tétrachloro-1-propène (HCO-1230zd), ou leurs mélanges pour la production du 1,3,3,3-tétrafluoropropène (HFO-1234ze).

Dans une deuxième réalisation, on diminue la teneur en fluor du composé hydrocarbure en déshydrofluorant ledit composé hydrocarbure en présence de ladite composition solide, ledit composé hydrocarbure étant un composé hydrocarbure fluoré.

Les composés hydrocarbures fluorés convenant comme matières premières au procédé de déshydrofluoration de cette invention sont typiquement saturés. On peut diminuer la teneur en fluor des composés saturés de formule (I) définie à la revendication 1 en présence d'un catalyseur étant une composition solide comprenant au moins un composant contenant de l'oxyfluorure ou du fluorure de chrome de formule brute CrₓM(₁₋ₓ)OᵣFₛ, où 2r+s est supérieur ou égal à 2,9 et inférieur à 6, M est Zn, x vaut entre 0,9 et 0,999, s est supérieur à 0 et inférieur ou égal à 6 et r est supérieur ou égal à 0 et inférieur à 3, ladite composition solide est amorphe.

Préférentiellement, le composant contenant de l'oxyfluorure ou du fluorure de chrome peut être de formule brute CrₓM(₁₋ₓ)OᵣFₛ, où r est supérieur ou égal à 0 et inférieur à 2,62, s est supérieur ou égal à [(0,16 r) + 0,52)]/0,92 à condition que s soit supérieur ou égal à 0,76 et inférieur à 6, plus préférentiellement le composant contenant de l'oxyfluorure ou du fluorure de chrome est de formule brute CrₓM(₁₋ₓ)OᵣFₛ, où r est supérieur ou égal à 0 et inférieur à 2,43, s est supérieur ou égal à [(0,24 r) + 0,78)]/0,88 à condition que s soit supérieur ou égal à 1,14 et inférieur à 6, idéalement le composant contenant de l'oxyfluorure ou du fluorure de chrome est de formule brute CrₓM(₁₋ₓ)OᵣFₛ où r est supérieur ou égal à 0 et inférieur à 2,24, s est supérieur ou égal à [(0,32 r) + 1,04)]/0,84 à condition que s soit supérieur ou égal à 1,52 et inférieur à 6, encore plus idéalement le composé contenant de l'oxyfluorure ou du fluorure de chrome est de formule brute CrₓM(₁₋ₓ)OᵣFₛ, où r est supérieur ou égal à 0 et inférieur à 2,05, s est supérieur ou égal à [(0,4 r) + 1,3)]/0,8 à condition que s soit supérieur ou égal à 1,90 et inférieur à 6, en particulier le composé contenant de l'oxyfluorure ou du fluorure de chrome est de formule brute CrₓM(₁₋ₓ)OᵣFₛ, où r est supérieur ou égal à 0 et inférieur à 1,75, s est supérieur ou égal à [(0,56 r) + 1,82)]/0,72 à condition que s soit supérieur ou égal à 2,50 et inférieur à 6. M est du zinc. Par conséquent, le composant contenant de l'oxyfluorure ou du fluorure de chrome est de formule brute CrₓZn(₁₋ₓ)OᵣFₛ, où x vaut entre 0,9 et 0,999, plus préférentiellement entre 0,94 et 0,99 ; avec r et s comme définis plus haut.

Dans une réalisation préférentielle, la composition solide contient un support choisi dans le groupe constitué de charbon actif, alumine et fluorure d'aluminium. La composition solide a une surface spécifique entre 15 et 45 m²/g.

En particulier, le composant contenant de l'oxyfluorure ou du fluorure de chrome est de formule brute CrₓM(₁₋ₓ)OᵣFₛ, où r est supérieur ou égal à 0 et inférieur à 2,05, s est supérieur ou égal à [(0,4 r) + 1,3)]/0,8 à condition que s soit supérieur ou égal à 1,90 et inférieur à 6, de préférence de formule brute CrₓM(₁₋ₓ)OᵣFₛ, où r est supérieur ou égal à 0 et inférieur à 1,75, s est supérieur ou égal à [(0,56 r) + 1,82)]/0,72 à condition que s soit supérieur ou égal à 2,50 et inférieur à 6, et une surface spécifique entre 15 et 45 m²/g. M est du zinc. Par conséquent, le composant contenant de l'oxyfluorure ou du fluorure de chrome peut être de formule brute CrₓZn(₁₋ₓ)OᵣFₛ, où x vaut entre 0,9 et 0,999, plus préférentiellement entre 0,94 et 0,99 ; avec r et s comme défini ci-dessus et une surface spécifique entre 15 et 45 m²/g.

Le procédé selon la deuxième réalisation peut être mené dans un réacteur comprenant un lit catalytique contenant un catalyseur et suivant les conditions opératoires suivantes :
- un rapport molaire HF/composé hydrocarbure entre 1:1 et 150:1, de préférence entre 2:1 et 125:1, plus préférentiellement entre 3:1 et 100:1 ;
- un temps de contact entre 1 et 100 s, de préférence entre 2 et 75 s, en particulier entre 3 et 50 s;
- une pression entre la pression atmosphérique et 20 bar, de préférence entre 2 et 18 bar, plus préférentiellement entre 3 et 15 bar ;
- une température (du lit catalytique) entre 200 et 450°C, de préférence entre 250 et 400°C, plus préférentiellement entre 280°C et 380°C.

Le procédé peut être mené à bien sur une durée comprise entre 10 et 8000 h, de préférence entre 50 et 5000 h, plus préférentiellement entre 70 et 1000 h.

On peut ajouter un oxydant, comme l'oxygène ou le chlore, en cours de procédé. Le rapport molaire de l'oxydant sur le composé hydrocarbure peut être entre 0,005 et 2, de préférence entre 0,01 et 1,5. L'oxydant peut être de l'oxygène pur, de l'air ou un mélange d'oxygène et d'azote.

Le procédé est mené typiquement dans un réacteur tubulaire. Le réacteur et ses lignes d'alimentation associées, lignes d'effluent et appareils associés doivent être construits dans des matériaux résistants au chlorure d'hydrogène. Les matériaux de construction typiques, bien connus en l'état de l'art de la fluoration, incluent les aciers inox, en particulier de type austénitique, les alliages bien connus à haute teneur en nickel, comme les alliages nickel-cuivre Monel^{®}, les alliages à base de nickel Hastelloy^{®} et les alliages nickel-chrome Inconel^{®}.

Le produit de la réaction de déshydrofluoration consiste en HF et en le composé hydrocarbure insaturé fluoré résultant de la perte de HF par le réactif initial. Des exemples spécifiques de réactions de déshydrofluoration en phase gazeuse qui peuvent être effectuées en utilisant les catalyseurs de cette invention incluent la conversion du 1,1,1,2-tétrafluoropropane (CH₃CHFCF₃ ou HFC-254eb) en 1,1,1-trifluoropropène (CH₂=CHCF₃ ou HFO-1243zf), la conversion du 1,1,1,3,3-pentafluoropropane (CHF₂CH₂CF₃ ou HFC-245fa) en 1,3,3,3-tétrafluoropropène (CHF=CHCF₃ ou HFO-1234ze), la conversion du 1,1,1,2,3,3-hexafluoropropane (CHF₂CHFCF₃ ou HFC-236ea) en 1,2,3,3,3-pentafluoropropène (CHF=CFCF₃ ou HFO-1225ye), la conversion du 1,1,1,2,3,3-hexafluoropropane (CF₃CF₂CFH₂ ou HFC-236cb) en 1,2,3,3,3-pentafluoropropène (CHF=CFCF₃ ou HFO-1225ye), la conversion du 1,1,1,2,2-pentafluoropropane (CF₃CF₂CH₃ ou HFC-245cb) en 2,3,3,3-tétrafluoropropène (CF₃CF=CH₂ ou HFO-1234yf) et la conversion du 1,1,1,2,3-pentafluoropropane (CF₃CHFCH₂F ou HFC-245eb) en 2,3,3,3-tétrafluoropropène (CF₃CF=CH₂ ou HFO-1234yf).

En particulier, le composé hydrocarbure halogéné est du 1,1,1,2,2-pentafluoropropane (HFC-245cb) pour la production of 2,3,3,3-tétrafluoropropène (HFO-1234yf). Sinon, le composé hydrocarbure halogéné est du 1,1,1,3,3-pentafluoropropane (HFC-245fa) pour la production de 1,3,3,3-tétrafluoropropène (HFO-1234ze).

Dans les procédés selon la première et la deuxième réalisation, la réaction dudit composé hydrocarbure avec du fluorure d'hydrogène peut être effectuée en présence d'oxygène ou de chlore.

Dans une troisième réalisation, on modifie la distribution en fluor dans le composé hydrocarbure en isomérisant ledit composé hydrocarbure en présence de ladite composition solide, ledit composé hydrocarbure étant un composé hydrocarbure fluoré.

Dans une quatrième réalisation, on modifie la distribution en fluor dans le composé hydrocarbure en dismutant ledit composé hydrocarbure en phase gazeuse en présence de ladite composition solide, ledit composé hydrocarbure étant un composé hydrocarbure chlorofluoré.

Les procédés d'isomérisation et de dismutation des troisième et quatrième réalisations sont menées à bien en phase vapeur en présence d'un catalyseur étant une composition solide comprenant au moins un composant contenant de l'oxyfluorure ou du fluorure de chrome de formule brute CrₓM(₁₋ₓ)OᵣFₛ, où 2r+s est supérieur ou égal à 2,9 et inférieur à 6, M est Zn, x vaut entre 0,9 et 0,999, s est supérieur à 0 et inférieur ou égal à 6 et r est supérieur ou égal à 0 et inférieur à 3.

Préférentiellement, le composant contenant de l'oxyfluorure ou du fluorure de chrome peut être de formule brute CrₓM(₁₋ₓ)OᵣFₛ, où r est supérieur ou égal à 0 et inférieur à 2,62, s est supérieur ou égal à [(0,16 r) + 0,52)]/0,92 à condition que s soit supérieur ou égal à 0,76 et inférieur à 6, plus préférentiellement le composant contenant de l'oxyfluorure ou du fluorure de chrome est de formule brute CrₓM(₁₋ₓ)OᵣFₛ, où r est supérieur ou égal à 0 et inférieur à 2,43, s est supérieur ou égal à [(0,24 r) + 0,78)]/0,88 à condition que s soit supérieur ou égal à 1,14 et inférieur à 6, idéalement le composant contenant de l'oxyfluorure ou du fluorure de chrome est de formule brute CrₓM(₁₋ₓ)OᵣFₛ, où r est supérieur ou égal à 0 et inférieur à 2,24, s est supérieur ou égal à [(0,32 r) + 1,04)]/0,84 à condition que s soit supérieur ou égal à 1,52 et inférieur à 6, encore plus idéalement le composé contenant de l'oxyfluorure ou du fluorure de chrome est de formule brute CrₓM(₁₋ₓ)OᵣFₛ, où r est supérieur ou égal à 0 et inférieur à 2,05, s est supérieur ou égal à [(0,4 r) + 1,3)]/0,8 à condition que s soit supérieur ou égal à 1,90 et inférieur à 6, en particulier le composé contenant de l'oxyfluorure ou du fluorure de chrome est de formule brute CrₓM(₁₋ₓ)OᵣFₛ, où r est supérieur ou égal à 0 et inférieur à 1,75, s est supérieur ou égal à [(0,56 r) + 1,82)]/0,72 à condition que s soit supérieur ou égal à 2,50 et inférieur à 6. M est du zinc. le composant contenant de l'oxyfluorure ou du fluorure de chrome peut être de formule brute CrₓZn(₁₋ₓ)OᵣFₛ, où x vaut entre 0,9 et 0,999, plus préférentiellement entre 0,94 et 0,99 ; avec r et s comme définis plus haut.

Dans une réalisation préférentielle, la composition solide comprend un support choisi dans le groupe constitué de charbon actif, alumine et fluorure d'aluminium. De préférence, la composition solide a une surface spécifique entre 15 à 45 m²/g.

En particulier, le composant contenant de l'oxyfluorure ou du fluorure de chrome est de formule brute CrₓM(₁₋ₓ)OᵣFₛ, où r est supérieur ou égal à 0 et inférieur à 2,05, s est supérieur ou égal à [(0,4 r) + 1,3)]/0,8 à condition que s soit supérieur ou égal à 1,90 et inférieur à 6, de préférence de formule brute CrₓM(₁₋ₓ)OᵣFₛ, où r est supérieur ou égal à 0 et inférieur à 1,75, s est supérieur ou égal à [(0,56 r) + 1,82)]/0,72 à condition que s soit supérieur ou égal à 2,50 et inférieur à 6, et une surface spécifique entre 15 à 45 m²/g. M est du zinc. Par conséquent, le composant contenant de l'oxyfluorure ou du fluorure de chrome est de formule brute CrₓZn(₁₋ₓ)OᵣFₛ, où x vaut entre 0,9 et 0,999, plus préférentiellement entre 0,94 et 0,99 ; avec r et s comme définis plus haut et avec une surface spécifique entre 15 à 45 m²/g.

Les composés hydrocarbures fluorés adéquats comme matières premières pour les procédés d'isomérisation et de dismutation peuvent être saturés ou insaturés.

On modifie la distribution de fluor d'un composé hydrocarbure fluoré en réarrangeant les substituants H, Br, Cl et F dans la molécule (typiquement un arrangement thermodynamiquement préférentiel) tout en maintenant le même nombre de substituants H, Br, Cl et F, respectivement. Dans la présente, ce procédé est appelé isomérisation.

On modifie la distribution de fluor d'un composé hydrocarbure fluoré en échangeant au moins un substituant F de la matière première hydrocarbure halogéné avec au moins un substituant H, Br et/ou Cl d'une autre molécule de la matière première hydrocarbure halogéné, de manière à donner la formation d'un ou plusieurs composés hydrocarbures halogénés ayant une teneur en fluor réduite par rapport à la matière première hydrocarbure halogéné et un ou plusieurs composés hydrocarbures halogénés ayant une teneur en fluor accrue par rapport à la matière première hydrocarbure halogéné. Dans la présente, ce procédé est appelé dismutation.

Les réactions d'isomérisation et de disputation peuvent survenir simultanément.

Que l'on effectue une isomérisation, une dismutation ou à la fois une isomérisation et une dismutation, on peut modifier la distribution en fluor de composés saturés et/ou de composés insaturés de formule (I) définie à la revendication 1 en présence d'un catalyseur comme dévoilé plus haut.

Les procédés d'isomérisation et de dismutation sont typiquement menés à bien à des températures entre environ 100°C et 500°C, de préférence entre environ 150°C et environ 400°C. La durée de mise en contact dans le réacteur est typiquement d'environ 1 à environ 120 s, de préférence d'environ 5 à environ 60 s. Les réactions d'isomérisation et de dismutation peuvent être menées à bien en présence d'un gaz inerte, comme l'hélium, l'argon ou l'azote, bien que ce ne soit pas préférentiel. Les réactions d'isomérisation et de dismutation peuvent être menées à bien en présence de HF et HCl.

Préférentiellement, les procédés d'isomérisation peuvent être effectués en utilisant le présent catalyseur et incluent la conversion du 1,3-dichloro-1,2,2,3,3-pentafluoropropane (CHClFCF₂CF₂Cl ou HCFC-225cb) en 1,1-dichloro-2,2,3,3,3-pentafluoropropane (CHCl₂CF₂CF₃ ou HCFC-225ca), la conversion du 2,2-dichloro-1,1,1,3,3-pentafluoropropane (CHF₂CCl₂CF₃ ou HCFC-225aa) en 1,1-dichloro-2,2,3,3,3-pentafluoropropane (CHCl₂CF₂CF₃ ou HCFC-225ca), la conversion du 1,1,1,2,3-pentafluoropropane (CF₃CHFCH₂F ou HFC-245eb) en 1,1,1,2,2-pentafluoropropane (CF₃CF₂CH₃ ou HFC-245cb), la conversion du 1,1,1,3,3,pentafluoropropane (CHF₂CH₂CF₃ ou HFC-245fa) en 1,1,1,2,3-pentafluoropropane (CF₃CHFCH₂F ou HFC-245eb), la conversion du 1,3,3,3-tétrafluoropropène (CHF=CHCF₃ ou HFO-1234ze) en 2,3,3,3-tétrafluoropropène (CF₃CF=CH₂ ou HFO-1234yf), la conversion du 1,1,3,3-tétrafluoropropène (CF₂=CHCHF₂ ou HFO-1234zc) en 1,3,3,3-tétrafluoropropéne (CHF=CHCF₃ ou HFO-1234ze), la conversion du 1-chloro-3,3,3-trifluoro-1-propène (CHCl=CHCF₃ ou HCFO-1233zd) en 2-chloro-3,3,3-trifluoro-1-propène (CH₂=CClCF₃ ou HCFO-1233xf) et la conversion de l'isomère (Z) des hydrochlorofluorooléfines en isomère (E) des hydrochlorofluorooléfines.

En particulier, les isomères (Z) d'hydrochlorofluorooléfines sont les isomères (Z) des hydrochlorofluoropropènes. Des exemples spécifiques incluent la conversion du (Z)-1-chloro-3,3,3-trifluoro-1-propène (CHCl=CHCF₃ ou HCFO-1233zd(Z)) en (E)-1-chloro-3,3,3-trifluoro-1-propène (CHCl=CHCF₃ ou HCFO-1233zd(E)), la conversion du (Z)-1,3,3,3-tétrafluoropropène (CHF=CHCF₃ ou HFO-1234ze(Z)) en (E)-1,3,3,3-tétrafluoropropène (CHF=CHCF₃ ou HFO-1234ze(E)), la conversion du (Z)-1,2,3,3,3-pentafluoropropène (CHF=CFCF₃ ou HFO-1225ye(Z)) en (E)-1,2,3,3,3-pentafluoropropène (CHF=CFCF₃ ou HFO-1225ye(E)).

Préférentiellement, les procédés de dismutation peuvent être effectués en utilisant le présent catalyseur et incluent la conversion du 1,1,3-trichloro-2,2,3,3-tétrafluoropropane (CHCl₂CF₂CF₂Cl ou HCFC-224ca) en 1,1-dichloro-2,2,3,3,3-pentafluoropropane (CHCl₂CF₂CF₃ ou HCFC-225ca) et 1,1,3,3-tétrachloro-1,2,2-trifluoropropane (CHCl₂CF₂CCl₂F ou HCFC-223ca), la conversion du 1,1,1,3-tétrafluoro-3-chloropropane (CF₃CH₂CHClF ou HCFC-244fa) en 1,1,1,3,3-pentafluoropropane (CHF₂CH₂CF₃ ou HFC-245fa) et en 1,1,1-trifluoro-3,3-dichloropropane (CF₃CH₂CHCl₂ ou HCFC-243fa), la conversion du 1,1,2,3-tétrafluoro-1-chloropropane (CF₂ClCHFCH₂F ou HCFC-244ec) en 1,1,1,2,3-pentafluoropropane (CF₃CHFCH₂F ou HFC-245eb) et en 1,2,3-trifluoro-1,1-dichloropropane (CFCl₂CHFCH₂F ou HCFC-243ed), la conversion du 1,1,2,2-tétrafluoro-1-chloropropane (CF₂ClCF₂CH₃ ou HCFC-244cc) en 1,1,1,2,2-pentafluoropropane (CF₃CF₂CH₃ ou HFC-245cb) et 1,2,2-trifluoro-1,1-dichloropropane (CFCl₂CF₂CH₃ ou HCFC-243cc), la conversion du 3-chloro-2,3,3-trifluoro-1-propène (CH₂=CFCClF₂ ou HCFO-1233yf) en 2,3,3,3-tétrafluoropropène (CF₃CF=CH₂ ou HFO-1234yf) et en 3,3-dichloro-2,3-difluoro-1-propène (CH₂=CFCFCl₂ ou HCFO-1232yf) et la conversion du 3-chloro-1,3,3-trifluoro-1-propène (CHF=CHCClF₂ ou HCFO-1233ze) en 1,3,3,3-tétrafluoropropène (CHF=CHCF₃ ou HFO-1234ze) et 3,3-dichloro-1,3-difluoro-1-propène (CHF=CHCCl₂F ou HCFO-1232ze).

Dans une cinquième réalisation, on diminue la teneur en fluor du composé hydrocarbure en faisant réagir ledit composé hydrocarbure avec du chlorure d'hydrogène en phase gazeuse en présence de ladite composition solide, ledit composé hydrocarbure étant un composé hydrocarbure halogéné. Ce procédé est effectué en présence d'un catalyseur étant une composition solide comprenant au moins un composant contenant de l'oxyfluorure ou du fluorure de chrome de formule brute CrₓM(₁₋ₓ)OᵣFₛ, où 2r+s est supérieur ou égal à 2,9 et inférieur à 6, M est Zn, x vaut entre 0,9 et 0,999, s est supérieur à 0 et inférieur ou égal à 6 et r est supérieur ou égal à 0 et inférieur à 3, ladite composition solide est amorphe.

Préférentiellement, le composant contenant de l'oxyfluorure ou du fluorure de chrome peut être de formule brute CrₓM(₁₋ₓ)OᵣFₛ, où r est supérieur ou égal à 0 et inférieur à 2,62, s est supérieur ou égal à [(0,16 r) + 0,52)]/0,92 à condition que s soit supérieur ou égal à 0,76 et inférieur à 6, plus préférentiellement le composant contenant de l'oxyfluorure ou du fluorure de chrome est de formule brute CrₓM(₁₋ₓ)OᵣFₛ, où r est supérieur ou égal à 0 et inférieur à 2,43, s est supérieur ou égal à [(0,24 r) + 0,78)]/0,88 à condition que s soit supérieur ou égal à 1,14 et inférieur à 6, idéalement le composant contenant de l'oxyfluorure ou du fluorure de chrome est de formule brute CrₓM(₁₋ₓ)OᵣFₛ, où r est supérieur ou égal à 0 et inférieur à 2,24, s est supérieur ou égal à [(0,32 r) + 1,04)]/0,84 à condition que s soit supérieur ou égal à 1,52 et inférieur à 6, encore plus idéalement le composé contenant de l'oxyfluorure ou du fluorure de chrome est de formule brute CrₓM(₁₋ₓ)OᵣFₛ, où r est supérieur ou égal à 0 et inférieur à 2,05, s est supérieur ou égal à [(0,4 r) + 1,3)]/0,8 à condition que s soit supérieur ou égal à 1,90 et inférieur à 6, en particulier le composé contenant de l'oxyfluorure ou du fluorure de chrome est de formule brute CrₓM(₁₋ₓ)OᵣFₛ, où r est supérieur ou égal à 0 et inférieur à 1,75, s est supérieur ou égal à [(0,56 r) + 1,82)]/0,72 à condition que s soit supérieur ou égal à 2,50 et inférieur à 6. le composant contenant de l'oxyfluorure ou du fluorure de chrome est de formule brute CrₓZn(₁₋ₓ)OᵣFₛ, où x vaut entre 0,9 et 0,999, plus préférentiellement entre 0,94 et 0,99 ; avec r et s comme définis plus haut.

Dans une réalisation préférentielle, la composition solide comprend un support choisi dans le groupe constitué de charbon actif, alumine et fluorure d'aluminium. De préférence, la composition solide a une surface spécifique entre 15 à 45 m²/g.

En particulier, le composant contenant de l'oxyfluorure ou du fluorure de chrome est de formule brute CrₓM(₁₋ₓ)OᵣFₛ, où r est supérieur ou égal à 0 et inférieur à 2,05, s est supérieur ou égal à [(0,4 r) + 1,3)]/0,8 à condition que s soit supérieur ou égal à 1,90 et inférieur à 6, de préférence de formule brute CrₓM(₁₋ₓ)OᵣFₛ, où r est supérieur ou égal à 0 et inférieur à 1,75, s est supérieur ou égal à [(0,56 r) + 1,82)]/0,72 à condition que s soit supérieur ou égal à 2,50 et inférieur à 6, et une surface spécifique entre 15 et 45 m²/g. le composant contenant de l'oxyfluorure ou du fluorure de chrome est de formule brute CrₓZn(₁₋ₓ)OᵣFₛ, où x vaut entre 0,9 et 0,999, plus préférentiellement entre 0,94 et 0,99 ; avec r et s comme définis plus haut et avec une surface spécifique entre 15 et 45 m²/g.

Les composés hydrocarbures fluorés adéquats comme matières premières pour le procédé de cette réalisation peuvent être saturés ou insaturés. Les composés hydrocarbures saturés halogénés adéquats pour les procédés de chlorodéfluoration selon cette invention incluent ceux de formule (I) définie à la revendication 1. Les composés hydrocarbures insaturés halogénés adéquats pour les procédés de chlorodéfluoration selon cette invention incluent ceux de formule (I) définie à la revendication 1.

Les réactions de chlorodéfluoration sont menées typiquement à des températures d'environ 250°C à 450°C, de préférence d'environ 300°C à environ 400°C. La durée de mise en contact dans le réacteur est typiquement d'environ 1 à environ 120 s. Bien sûr, des durées de contact d'environ 5 à environ 60 s sont possibles. Les réactions sont idéalement menées à pression atmosphérique ou plus.

Les chlorodéfluorations impliquant des hydrocarbures halogénés saturés sont particulièrement dignes d'intérêt. Le rapport molaire de HCl au composé hydrocarbure halogéné saturé se situe typiquement entre environ 1:1 et environ 100:1, de préférence d'environ 3:1 à environ 50:1, et idéalement d'environ 4:1 à environ 30:1. En général, avec une composition catalytique donnée, plus haute est la température, plus longue est la durée de contact, plus grand est le rapport molaire de HCl au composé hydrocarbure halogéné saturé, et plus importante est la conversion des composés à faible teneur en fluor. On peut équilibrer les variables ci-dessus les unes par rapport aux autres pour maximiser la formation de produits chlorés.

Le produit des réactions de chlorodéfluoration comprend typiquement du HCl et du HF non-réagit, de la matière première non-convertie et des composés hydrocarbures saturés halogénés ayant une teneur en fluor plus faible que la matière première par suite de la substitution d'un ou plusieurs substituants fluor par du chlore.

On peut séparer les produits réactionnels obtenus par les procédés détaillés dans l'une quelconque des cinq premières réalisations par des techniques conventionnelles, comme avec des combinaisons incluant, non-limitativement, lavage, décantation ou distillation. Certains des produits des diverses réalisations de cette invention peuvent former un ou plusieurs azéotropes les uns avec les autres ou avec HF.

Les procédés dévoilés dans la présente invention peuvent inclure, en outre, l'étape de régénération de ladite composition solide en présence d'un flux de régénération comprenant un flux d'air/oxydant. L'oxydant peut être de l'oxygène, de l'air, un mélange d'oxygène et d'azote, du chlore ou un mélange de chlore et d'azote. Quand la régénération est effectuée avec de l'air ou un mélange d'oxygène et d'azote, la proportion d'oxygène peut aller de 5 à 100 % en moles rapporté au mélange d'oxygène et d'azote.

L'étape de régénération peut être effectuée en présence d'un flux de régénération contenant (a) de l'oxygène ou de l'air ou un mélange oxygène/azote ou du chlore et (b) HF.

Avantageusement, le flux de régénération contiendra au moins 1% en moles d'oxygène rapporté au flux total de régénération. La proportion d'oxygène peut aller de 2 à 98 % en moles rapporté à la quantité totale exprimée en moles d'oxygène et HF, et de 20 à 100 % en moles rapporté à la quantité totale exprimée en moles d'oxygène et d'azote.

L'étape de régénération est menée à bien à une température de 250 à 500°C, de préférence de 300 à 450°C, plus préférentiellement de 350 à 400°C. L'étape de régénération peut être menée à bien avec une durée de contact de 1 à 200 s, de préférence de 1 à 150 s, plus préférentiellement de 5 à 100 s, et pour une durée de 1 à 1500 h, de préférence de 2 à 1000 h, plus préférentiellement de 4 à 500 h, idéalement de 10 à 200 h et en particulier de 15 à 150 h. L'étape de régénération peut être menée à bien sous une pression allant de la pression atmosphérique à 20 bar. En particulier, l'étape de régénération peut être menée à bien à une température de 250 à 500°C, avec une durée de contact de 1 à 200 s, pendant 10 à 200 h et sous une pression entre la pression atmosphérique et 20 bar.

Les procédés dévoilés dans la présente invention peuvent comprendre, en outre, l'étape d'activation de ladite composition solide en présence d'un flux d'air/oxydant.

Avant utilisation, il est préférable que le catalyseur soit soumis à une étape d'activation avec de l'air, de l'oxygène ou du chlore et/ou HF. Par exemple, le catalyseur est préférentiellement soumis à une activation avec de l'air ou de l'oxygène, et HF à une température entre 100 et 500°C, de préférence entre 250 et 500°C et en particulier entre 300 et 400°C. La durée d'activation est préférentiellement de 1 à 200 h et en particulier de 1 à 50 h. Cette activation peut être suivie d'une étape finale d'activation de fluoration en présence d'un oxydant, HF et de composés hydrocarbures. Le rapport molaire HF/composé hydrocarbure va de 2 à 40, et le rapport molaire oxydant/composé hydrocarbure va de 0,04 à 25. La température de l'étape finale d'activation de fluoration peut aller de 300 à 400°C, de préférence pour une durée de 6 à 100 h.

Dans une sixième réalisation, on augmente la teneur en fluor d'un premier composé hydrocarbure en faisant réagir ledit premier composé hydrocarbure avec du fluorure d'hydrogène en phase gazeuse en présence d'une composition solide, le premier composé hydrocarbure étant un hydrocarbure halogéné saturé ou un hydrocarbure halogéné insaturé ou un hydrocarbure insaturé et on diminue la teneur en fluor d'un deuxième composé hydrocarbure en déshydrofluorant ledit deuxième composé hydrocarbure en présence de ladite composition solide, ledit deuxième composé hydrocarbure étant un composé hydrocarbure fluoré. Le premier composé hydrocarbure étant un hydrocarbure halogéné saturé ou un hydrocarbure halogéné insaturé ou un hydrocarbure insaturé est défini ci-dessus en référence à la première réalisation. Le deuxième composé hydrocarbure est défini ci-dessus en référence à la deuxième réalisation. La fluoration du premier composé hydrocarbure et la déshydrofluoration du deuxième composé hydrocarbure sont préférentiellement effectuées simultanément.

La composition solide comprend au moins un composant contenant de l'oxyfluorure ou du fluorure de chrome de formule brute CrₓM(₁₋ₓ)OᵣFₛ, où 2r+s est supérieur ou égal à 2,9 et inférieur à 6, M est Zn, x vaut entre 0,9 et 0,999, s est supérieur à 0 et inférieur ou égal à 6 and r est supérieur ou égal à 0 et inférieur à 3, ladite composition solide est amorphe.

Préférentiellement, le composant contenant de l'oxyfluorure ou du fluorure de chrome peut être de formule brute CrₓM(₁₋ₓ)OᵣFₛ, où r est supérieur ou égal à 0 et inférieur à 2,62, s est supérieur ou égal à [(0,16 r) + 0,52)]/0,92 à condition que s soit supérieur ou égal à 0,76 et inférieur à 6, plus préférentiellement le composant contenant de l'oxyfluorure ou du fluorure de chrome est de formule brute CrₓM(₁₋ₓ)OᵣFₛ, où r est supérieur ou égal à 0 et inférieur à 2,43, s est supérieur ou égal à [(0,24 r) + 0,78)]/0,88 à condition que s soit supérieur ou égal à 1,14 et inférieur à 6, idéalement le composant contenant de l'oxyfluorure ou du fluorure de chrome est de formule brute CrₓM(₁₋ₓ)OᵣFₛ, où r est supérieur ou égal à 0 et inférieur à 2,24, s est supérieur ou égal à [(0,32 r) + 1,04)]/0,84 à condition que s soit supérieur ou égal à 1,52 et inférieur à 6, encore plus idéalement le composé contenant de l'oxyfluorure ou du fluorure de chrome est de formule brute CrₓM(₁₋ₓ)OᵣFₛ, où r est supérieur ou égal à 0 et inférieur à 2,05, s est supérieur ou égal à [(0,4 r) + 1,3)]/0,8 à condition que s soit supérieur ou égal à 1,90 et inférieur à 6, en particulier le composé contenant de l'oxyfluorure ou du fluorure de chrome est de formule brute CrₓM(₁₋ₓ)OᵣFₛ, où r est supérieur ou égal à 0 et inférieur à 1,75, s est supérieur ou égal à [(0,56 r) + 1,82)]/0,72 à condition que s soit supérieur ou égal à 2,50 et inférieur à 6. Le composant contenant de l'oxyfluorure ou du fluorure de chrome est de formule brute CrₓZn(₁₋ₓ)OᵣFₛ, où x vaut entre et 0,999, plus préférentiellement entre 0,94 et 0,99 ; avec r et s comme définis plus haut.

Dans une réalisation préférentielle, la composition solide comprend un support choisi dans le groupe constitué de charbon actif, alumine et fluorure d'aluminium. La composition solide a une surface spécifique de 15 à 45 m²/g.

En particulier, le composant contenant de l'oxyfluorure ou du fluorure de chrome est de formule brute CrₓM(₁₋ₓ)OᵣFₛ, où r est supérieur ou égal à 0 et inférieur à 2,05, s est supérieur ou égal à [(0,4 r) + 1,3)]/0,8 à condition que s soit supérieur ou égal à 1,90 et inférieur à 6, de préférence de formule brute CrₓM(₁₋ₓ)OᵣFₛ, où r est supérieur ou égal à 0 et inférieur à 1,75, s est supérieur ou égal à [(0,56 r) + 1,82)]/0,72 à condition que s soit supérieur ou égal à 2,50 et inférieur à 6, et avec une surface spécifique entre 15 et 45 m²/g. M est du zinc. Par conséquent, le composant contenant de l'oxyfluorure ou du fluorure de chrome est de formule brute CrₓZn(₁₋ₓ)OᵣFₛ, où x vaut entre 0,9 et 0,999, plus préférentiellement entre 0,94 et 0,99 ; avec r et s comme définis plus haut et avec une surface spécifique entre 15 et 45 m²/g.

Le procédé selon la sixième réalisation peut être mené dans un réacteur comprenant un lit catalytique contenant un catalyseur et suivant les conditions opératoires suivantes :
- un rapport molaire HF/composé hydrocarbure de 1:1 à 150:1, de préférence de 2:1 à 125:1, plus préférentiellement de 3:1 à 100:1 ;
- une durée de contact de 1 à 100 s, de préférence de 2 à 75 s, en particulier de 3 à 50 s ;
- une pression entre la pression atmosphérique et 20 bar, de préférence entre 2 et 18 bar, plus préférentiellement entre 3 et 15 bar ;
- une température (du lit catalytique) entre 200 et 450°C, de préférence entre 250 et 400°C, plus préférentiellement entre 280°C et 380°C.

Le procédé peut être mené à bien sur une durée comprise entre 10 et 8000 h, de préférence entre 50 et 5000 h, plus préférentiellement entre 70 et 1000 h.

On peut ajouter un oxydant, comme l'oxygène ou le chlore, en cours de procédé. Le rapport molaire de l'oxydant sur le composé hydrocarbure peut être entre 0,005 et 2, de préférence entre 0,01 et 1,5. L'oxydant peut être de l'oxygène pur, de l'air ou un mélange d'oxygène et d'azote.

Le procédé est mené typiquement dans un réacteur tubulaire. Le réacteur et ses lignes d'alimentation associées, lignes d'effluent et appareils associés doiventêtre construits dansdes matériaux résistants au fluorure d'hydrogène et au chlorure d'hydrogène. Les matériaux de construction typiques, bien connus en l'état de l'art de la fluoration, incluent les aciers inox, en particulier de type austénitique, les alliages bien connus à haute teneur en nickel, comme les alliages nickel-cuivre Monel^{®}, les alliages à base de nickel Hastelloy^{®} et les alliages nickel-chrome Inconel^{®}.

Les produits de la réaction sont ceux détaillés en référence à la première et la deuxième réalisation. En particulier, la composition solide est utile pour la conversion du 1,1,1,2,3-pentachloropropane (CCl₃CHClCH₂Cl ou HCC-240db) en 2-chloro-3,3,3-trifluoro-1-propène (CF₃CCl=CH₂ ou HCFO-1233xf) ou la conversion du 1,1,1,2,3-pentachloropropane (CCl₃CHClCH₂Cl ou HCC-240db) en 2,3,3,3-tétrafluoropropène (CF₃CF=CH₂ ou HFO-1234yf) ou la conversion du 2-chloro-3,3,3-trifluoro-1-propène (CF₃CCl=CH₂ ou HCFO-1233xf) en 2,3,3,3-tétrafluoropropène (CF₃CF=CH₂ ou HFO-1234yf) et la conversion du 1,1,1,2,2-pentafluoropropane (CF₃CF₂CH₃ ou HFC-245cb) en 2,3,3,3-tétrafluoropropène (CF₃CF=CH₂ ou HFO-1234yf) ou la conversion du 1,1,1,2,3-pentafluoropropane (CF₃CHFCH₂F ou HFC-245eb) en 2,3,3,3-tétrafluoropropène (CF₃CF=CH₂ ou HFO-1234yf). En particulier, la composition solide est utile pour la conversion du 2-chloro-3,3,3-trifluoro-1-propène (CF₃CCl=CH₂ ou HCFO-1233xf) en 2,3,3,3-tétrafluoropropène (CF₃CF=CH₂ ou HFO-1234yf) et la conversion du 1,1,1,2,2-pentafluoropropane (CF₃CF₂CH₃ ou HFC-245cb) en 2,3,3,3-tétrafluoropropène (CF₃CF=CH₂ ou HFO-1234yf).

Dans une septième réalisation, on augmente la teneur en fluor d'un premier composé hydrocarbure en faisant réagir ledit premier composé hydrocarbure avec du fluorure d'hydrogène en phase gazeuse en présence d'une composition solide, le premier composé hydrocarbure étant un hydrocarbure halogéné saturé ou un hydrocarbure halogéné insaturé ou un hydrocarbure insaturé et on diminue la teneur en chlore d'un deuxième composé hydrocarbure en déshydrochlorant ledit deuxième composé hydrocarbure en présence de ladite composition solide, ledit deuxième composé hydrocarbure étant un composé hydrocarbure fluoré.

Dans cette réalisation, la composition solide selon la présente invention est utile pour la conversion du 2-chloro-3,3,3-trifluoro-1-propène (CF₃CCl=CH₂ ou HCFO-1233xf) en 2,3,3,3-tétrafluoropropène (CF₃CF=CH₂ ou HFO-1234yf) et la conversion du 2-chloro-1,1,1,2-tétrafluoropropane (CF₃CFClCH₃ ou HCFC-244bb) en 2,3,3,3-tétrafluoropropène (CF₃CF=CH₂ ou HFO-1234yf).

La composition solide comprend au moins un composant contenant de l'oxyfluorure ou du fluorure de chrome de formule brute CrₓM(₁₋ₓ)OᵣFₛ, où 2r+s est supérieur ou égal à 2,9 et inférieur à 6, M est Zn, x vaut entre 0,9 et 0,999, s est supérieur à 0 et inférieur ou égal à 6 and r est supérieur ou égal à 0 et inférieur à 3, ladite composition solide est amorphe.

Préférentiellement, le composant contenant de l'oxyfluorure ou du fluorure de chrome peut être de formule brute CrₓM(₁₋ₓ)OᵣFₛ, où r est supérieur ou égal à 0 et inférieur à 2,62, s est supérieur ou égal à [(0,16 r) + 0,52)]/0,92 à condition que s soit supérieur ou égal à 0,76 et inférieur à 6, plus préférentiellement le composant contenant de l'oxyfluorure ou du fluorure de chrome est de formule brute CrₓM(₁₋ₓ)OᵣFₛ, où r est supérieur ou égal à 0 et inférieur à 2,43, s est supérieur ou égal à [(0,24 r) + 0,78)]/0,88 à condition que s soit supérieur ou égal à 1,14 et inférieur à 6, idéalement le composant contenant de l'oxyfluorure ou du fluorure de chrome est de formule brute CrₓM(₁₋ₓ)OᵣFₛ, où r est supérieur ou égal à 0 et inférieur à 2,24, s est supérieur ou égal à [(0,32 r) + 1,04)]/0,84 à condition que s soit supérieur ou égal à 1,52 et inférieur à 6, encore plus idéalement le composé contenant de l'oxyfluorure ou du fluorure de chrome est de formule brute CrₓM(₁₋ₓ)OᵣFₛ, où r est supérieur ou égal à 0 et inférieur à 2,05, s est supérieur ou égal à [(0,4 r) + 1,3)]/0,8 à condition que s soit supérieur ou égal à 1,90 et inférieur à 6, en particulier le composé contenant de l'oxyfluorure ou du fluorure de chrome est de formule brute CrₓM(₁₋ₓ)OᵣFₛ, où r est supérieur ou égal à 0 et inférieur à 1,75, s est supérieur ou égal à [(0,56r) + 1,82)]/0,72 à condition que s soit supérieur ou égal à 2,50 et inférieur à 6. le composant contenant de l'oxyfluorure ou du fluorure de chrome est de formule brute CrₓZn(₁₋ₓ)OᵣF, où x vaut 0,9 et 0,999, plus préférentiellement entre 0,94 et 0,99 ; avec r et s comme définis plus haut.

Dans une réalisation préférentielle, la composition solide comprend un support choisi dans le groupe constitué de charbon actif, alumine et fluorure d'aluminium. La composition solide a une surface spécifique de 15 à 45 m²/g.

En particulier, le composant contenant de l'oxyfluorure ou du fluorure de chrome est de formule brute CrₓM(₁₋ₓ)OᵣFₛ, où r est supérieur ou égal à 0 et inférieur à 2,05, s est supérieur ou égal à [(0,4 r) + 1,3)]/0,8 à condition que s soit supérieur ou égal à 1,90 et inférieur à 6, de préférence de formule brute CrₓM(₁₋ₓ)OᵣFₛ, où r est supérieur ou égal à 0 et inférieur à 1,75, s est supérieur ou égal à [(0,56 r) + 1,82)]/0,72 à condition que s soit supérieur ou égal à 2,50 et inférieur à 6, et une surface spécifique entre 15 et 45 m²/g.-M est du zinc. Par conséquent, le composant contenant de l'oxyfluorure ou du fluorure de chrome est de formule brute CrₓZn(₁₋ₓ)OᵣFₛ, où x vaut entre 0,9 et 0,999, plus préférentiellement entre 0,94 et 0,99 ; avec r et s comme définis plus haut et une surface spécifique entre 15 et 45 m²/g.

Le procédé selon la sixième réalisation peut être mené dans un réacteur comprenant un lit catalytique contenant un catalyseur et suivant les conditions opératoires suivantes :
- un rapport molaire HF/composé hydrocarbure de 1:1 à 150:1, de préférence de 2:1 à 125:1, plus préférentiellement de 3:1 à 100:1 ;
- une durée de contact de 1 à 100 s, de préférence de 2 à 75 s, en particulier de 3 à 50 s ;
- une pression entre la pression atmosphérique et 20 bar, de préférence entre 2 et 18 bar, plus préférentiellement entre 3 et 15 bar ;
- une température (du lit catalytique) entre 200 et 450°C, de préférence entre 250 et 400°C, plus préférentiellement entre 280°C et 380°C.

Le procédé peut être mené à bien sur une durée comprise entre 10 et 8000 h, de préférence entre 50 et 5000 h, plus préférentiellement entre 70 et 1000 h.

Il est décrit un procédé de préparation d'une composition catalytique solide utilisée dans le procédé selon la présente invention. Ledit procédé comprend :
(a) une étape réactionnelle comprenant la mise en contact d'un composant oxyde de chrome en présence de fluorure d'hydrogène et d'un composé hydrocarbure halogéné à une température de 200 à 450°C pendant au moins 100 h pour former un composant oxyde de chrome dédié,
(b) une étape de régénération de l'oxyde de chrome dédié en présence d'un flux gazeux d'un oxydant, de préférence de l'air, à une température de 250 à 500°C pendant au moins 1 h, de préférence au moins pour former un composant oxyde de chrome régénéré,
(c) la répétition au moins deux fois des étapes (a) et (b) avec l'oxyde chrome régénéré jusqu'à ce que ledit composant oxyde de chrome régénéré soit devenu un composant contenant de l'oxyfluorure ou du fluorure de chrome de formule brute CrₓM(₁₋ₓ)OᵣFₛ, dans lequel 2r+s est supérieur ou égal à 2,9 et inférieur à 6, M est Zn, x vaut entre 0,9 et 0,999, s est supérieur à 0 et inférieur ou égal à 6 et r est supérieur ou égal à 0 et inférieur à 3, ladite composition solide étant amorphe et présentant une surface spécifique entre 15 et 45 m2/g.

### Exemples

Procédé de quantification de la cristallinité :
On détermine la cristallinité en fonction du résultat d'une mesure de cristallographie aux rayons X. Spécifiquement, la cristallinité désigne le rapport déterminé par la comparaison entre la surface des pics de diffraction de tous les plans cristallins de l'échantillon standard avec celle du composant cible contenant de l'oxyfluorure ou du fluorure de chrome, chaque surface étant calculée à partir d'un schéma de diffraction obtenu par mesure de cristallographie aux rayons X dans les mêmes conditions. On a analysé des composants de cristallinité connue pour élaborer une courbe d'étalonnage, à partir de laquelle on peut déterminer la cristallinité du composant contenant de l'oxyfluorure ou du fluorure de chrome.

### Exemples 1 et 2 :

Fluoration du 1,1,1,2,3-pentachloropropane (CCl₃CHClCH₂Cl ou HCC-240db)

L'équipement consiste en deux réacteurs multitubulaires en série, chaque tube ayant un diamètre interne de 28 mm, fabriqué en alliage INCONEL^{®} alloy 600. La Figure 1 représente le procédé effectué dans les exemples 1 et 2. Le premier réacteur en phase gazeuse 3 est alimenté par du HCC-240db frais via la ligne 2, et facultativement en HF frais via la ligne 1. Le mélange réactionnel 4 qui quitte le réacteur contient HCl, HCFO-1233xf, HF non-réagit, HFO-1234yf et facultativement HFC-245cb. Ce flux réactionnel est séparé par distillation 5 en un premier flux 6 contenant HCl, HFO-1234yf, facultativement avec une petite quantité de HF et des quantités mineures de HFC-245cb et HFO-1233xf. On obtient un deuxième flux plus lourd 7 au fond de la colonne de distillation, qui contient HF, HCFO-1233xf, HFC-245cb. On peut séparer et purifier HFO-1234yf depuis le flux 6 en utilisant des procédés connus.

Le deuxième réacteur 10 est alimenté par le flux 9 qui consiste en le deuxième flux 7, facultativement avec HF frais 8. Le mélange réactionnel 11 qui quitte le réacteur contient HCl, HCFO-1233xf non-réagit, HF non-réagit, HFO-1234yf, HFC-245cb. Ce mélange réactionnel est envoyé directement au premier réacteur sans être soumis à aucune séparation.

Le catalyseur est une masse amorphe d'oxyde de chrome Cr₂O₃ activé par 2 % en poids de zinc, contenant environ 4 % de graphite. Le réacteur est équipé d'un contrôleur de pression et de température. En sortie du réacteur, les produits de la réaction sont lavés à travers un scrubber KOH pour éliminer les hydracides. On prélève plusieurs échantillons pour analyse en ligne par chromatographie en phase gazeuse : entrée du premier réacteur, sortie du premier réacteur, sortie du deuxième réacteur et produit final en tête de la colonne de distillation. Deux analyses CPG différentes sont nécessaires pour détecter la vaste gamme de produits possibles. L'analyse chromatographique est effectuée sur une colonne RTX 200 pour quantifier une large gamme de produits organiques, dimensions 105 m x 0,32 mm × 2 µm. La programmation de la température du four est la suivante : 40°C pendant 10 min, puis pente de 10°C/min jusqu'à 300°C. L'analyse chromatographique est effectuée sur une colonne Shincarbon 2 m x 1,8" pour une quantification et une meilleure séparation des produits les plus légers ainsi que des produits inertes (CO, CO₂, O₂). La programmation de la température du four est la suivante : 40°C pendant 10 min, puis pente de 10°C/min jusqu'à 300°C.

### Exemple 1 :

Le catalyseur a été préfluoré avant réaction. La préfluoration a été effectuée en effectuant une réaction de fluoration pendant 100 h dans les conditions suivantes : T = 350°C, P = 5 bar avec un rapport molaire HF/organique de 15 à 30, alternativement avec une étape de régénération effectuée avec de l'air à 360°C. On répète les étapes de réaction et régénération. Le catalyseur CrₓZn(₁₋ₓ)OᵣFₛ a été analysé avant utilisation : s = 1,7 et r = 0,7, x = 0,97 et la surface spécifique était d'environ 42 m²/g. Le catalyseur est amorphe. La réaction de fluoration est ensuite effectuée à T = 350°C sous une pression absolue de 5 bar. L'alimentation en HF frais était de 1,6 kg/h, et le flux de composé chloré était proche de 4 kg/h. Le débit de la boucle de recyclage était de 34 kg/h, ce qui donne une durée de contact sur le premier réacteur proche de 15 s. Le rapport molaire HF/organique est compris entre 15 et 20: il correspond au rapport entre HF et la somme des composés organiques. Les résultats sont reportés dans le Tableau 1 ci-dessous. On obtient de bonnes sélectivités tout au long de la manipulation.

### Exemple 2 :

Le catalyseur a été préfluoré avant réaction. La préfluoration a été effectuée en effectuant une réaction de fluoration pendant 840 h dans les conditions suivantes : T = 350°C, P = 5 bar avec un rapport molaire HF/organique de 15 à 30, alternativement avec une étape de régénération effectuée avec de l'air à 360°C. Le catalyseur CrₓZn(₁₋ₓ)OᵣFₛ a été analysé avant utilisation : s = 3,0 et r = 0,17, x = 0,98 et la surface spécifique était d'environ 22 m²/g. Le catalyseur est amorphe. La réaction de fluoration est ensuite effectuée à T = 350°C sous une pression absolue de 5 bar. L'alimentation en HF frais était de 1,7 kg/h, et le flux de composé chloré était proche de 3,9 kg/h. Le débit de la boucle de recyclage était de 34 kg/h, ce qui donne une durée de contact sur le premier réacteur proche de 15 s. Le rapport molaire HF/organique est compris entre 15 et 20 : il correspond au rapport entre HF et la somme des composés organiques. Les résultats sont reportés dans le Tableau 1 ci-dessous. On obtient de bonnes sélectivités tout au long de la manipulation.

**Tableau 1**

| Durée d'écoulement (h) | Conversion de HCFO-F1233xf dans le réacteur (10) Exemple 1 | Durée d'écoulement (h) | Conversion de HCFO-F1233xf dans le réacteur (10) Exemple 2 |
|---|---|---|---|
| 2,2 | 70,9 (%) | 15,0 | 73,1 (%) |
| 18,5 | 56,8 (%) | 90,8 | 69,9 (%) |
| 26,0 | 50,6 (%) | 153,1 | 66,7 (%) |
| 34,2 | 52,3 (%) | 235,0 | 62,1 (%) |
| 42,3 | 47,7 (%) | 315,9 | 57,2 (%) |
| 50,5 | 42,9 (%) | 389,0 | 50,9 (%) |
| 58,7 | 42,9 (%) | 421,4 | 47,8 (%) |
| 66,8 | 39,5 (%) | 453,9 | 42,7 (%) |
| 74,8 | 35,8 (%) | 495,0 | 37,1 (%) |

### Exemple 3 :

### Isomérisation du 1,1,1,2,3-pentafluoropropane (CF₃CHFCH₂F ou HFC-245eb)

Le catalyseur utilisé dans cet exemple était un catalyseur commercial de chrome amorphe en vrac. Le catalyseur a été préfluoré avant réaction. La préfluoration a été effectuée par une réaction de fluoration (R-CI + HF → R-F + HCl) pendant 1000 h dans les conditions suivantes : T = 350°C, P = 3 bar et HF/organique entre 20 et 30. Le catalyseur CrOᵣFₛ a été analysé avant utilisation : s = 1,9 et r = 0,54, la surface spécifique était d'environ 32 m²/g. Un réacteur à lit fixe Hastelloy C (3/4" x 16") a été chargé avec 23 cm³ de catalyseur commercial Cr préfluoré en vrac. Le catalyseur a été évalué sous pression atmosphérique à une température de 375°C en utilisant un mélange de HFC-245eb et d'azote (rapport molaire N₂/HFC-245eb = 1,4). Les conditions d'alimentation correspondent à une durée de contact de 9 s. Le produit organique obtenu depuis le réacteur a été lavé, séché et analysé par chromatographie en phase gazeuse. On a observé une excellente conversion de HFC-245eb avec une sélectivité prometteuse pour le 1,1,1,2,2-pentafluoropropane (CF₃CF₂CH₃ ou HFC-245cb).

### Exemple 4 :

### Déshydrofluoration du 1,1,1,3,3-pentafluoropropane (CHF₂CH₂CF₃ou HFC-245fa)

On a utilisé le catalyseur de l'exemple 3 pour déshydrofluorer HFC-245fa dans un réacteur similaire à celui décrit à l'exemple 3. 20 cm³ du catalyseur commercial de chrome préfluoré amorphe en vrac. Le catalyseur a été évalué sous pression atmosphérique à une température de 400°C en utilisant un mélange de HFC-245fa et d'air (égal à environ 3 % en volume d'oxygène rapporté au volume de HFC-245fa). Les conditions d'alimentation correspondent à une durée de contact de 40 s. La conversion de HFC-245fa a été supérieure à 90%, avec une excellente sélectivité pour le 1,3,3,3-tétrafluoropropène (CHF=CHCF₃ ou HFO-1234ze) (supérieure à 90%).

### Exemple 5 (comparatif) :

### Fluoration du 1,1,2-trichloroéthane (CHCl₂CH₂Cl ou HCC-140)

Le catalyseur utilisé dans cet exemple était un catalyseur commercial de chrome amorphe en vrac. Le catalyseur a été préfluoré avant réaction en utilisant un traitement avec HF pur à T = 350°C jusqu'à obtention du composé désiré. Le catalyseur CrOᵣFₛ a été analysé avant utilisation : s = 1,8 et r = 0,62, et la surface spécifique était d'environ 46 m²/g. Un réacteur monotubulaire en Inconel a été chargé avec 40 g de catalyseur Cr prétraité. Le réacteur a été monté à l'intérieur d'une chaudière, et la zone réactionnelle a été maintenue à 225°C. La pression a été maintenue à environ P = 11 bar. L'alimentation organique (CHCl₂CH₂Cl ou HCC-140), en fluorure d'hydrogène et en chlore a été introduire dans le réacteur dans un rapport molaire de 1:20:0,08. Le taux de HF était d'environ 1,8 mol/h et la durée de contact était d'environ 18 s. On a utilisé une CPG pour analyser l'effluent du réacteur. Le produit désiré 1-chloro-2,2-difluoroéthane (CH₂ClCF₂H ou HCFC-142) a été obtenu avec un rendement de 65 %.

## Revendications

1. Procédé de modification de la distribution en fluor dans un composé hydrocarbure de formule (I) CX(Y)₂-CX(Y)ₘ-CHₘXY où X et Y représentent indépendamment H, F ou Cl et m = 0 ou 1 avec au moins un parmi X ou Y qui est Cl ou F ; en présence d'un catalyseur, le catalyseur étant une composition solide contenant au moins un composant contenant de l'oxyfluorure ou du fluorure de chrome de formule brute CrₓM(₁₋ₓ)OᵣFₛ, où 2r+s est supérieur ou égal à 2,9 et inférieur à 6, M est Zn, x vaut de 0,9 à 0,999, s est supérieur à 0 et inférieur ou égal à 6 et r est supérieur ou égal à 0 et inférieur à 3, **caractérisé en ce que** ledit composant contenant de l'oxyfluorure ou du fluorure de chrome est amorphe, et **en ce que** la composition solide a une surface spécifique entre 15 et 45 m²/g.

2. Procédé selon l'une quelconque des revendications précédentes dans lequel ledit composant contenant de l'oxyfluorure ou du fluorure de chrome est de formule brute CrₓM(₁₋ₓ)OᵣFₛ avec M = Zn ; en particulier x vaut de 0,94 à 0,99.

3. Procédé selon la revendication précédente dans lequel le composé hydrocarbure est choisi dans le groupe constitué du tétrachloropropène, chlorotrifluoropropène, pentachloropropane, dichlorotrifluoropropane, trichlorodifluoropropane, tétrafluorochloropropane, tétrachlorofluoropropane, dichlorodifluoropropène, trichlorofluoropropène, pentafluoropropane et leurs mélanges ; de préférence, le composé hydrocarbure est choisi dans le groupe constitué du 2-chloro-3,3,3-trifluoro-1-propène (HFCO-1233xf), 2,3-dichloro-1,1,1-trifluoropropane (HCFC-243db), 1,1,1,2,3-pentachloropropane (HCC-240db), 1,1,2,2,3-pentachloropropane (HCC-240aa), 1,1,1,3,3-pentachloropropane (HCC-240fa), 1,1,2,3-tétrachloro-1-propène (HCO-1230xa), 2,3,3,3-tétrachloro-1-propène (HCO-1230xf), 1,1,3,3-tétrachloro-1-propène (HCO-1230za), 1,3,3,3-tétrachloro-1-propène (HCO-1230zd), 1,1,1,2,2-pentafluoropropane (HFC-245cb) et 1-chloro-3,3,3-trifluoro-1-propène (HCFO-1233zd).

4. Procédé selon l'une quelconque des revendications précédentes dans lequel on augmente la teneur en fluor du composé hydrocarbure en faisant réagir ledit composé hydrocarbure avec du fluorure d'hydrogène en phase gazeuse en présence de ladite composition solide, le composé hydrocarbure étant un hydrocarbure saturé halogéné ou un hydrocarbure insaturé halogéné ou un hydrocarbure insaturé.

5. Procédé selon l'une quelconque des revendications 1 à 3 dans lequel on diminue la teneur en fluor du composé hydrocarbure par déshydrofluoration dudit composé hydrocarbure en présence de ladite composition solide, ledit composé hydrocarbure étant un composé hydrocarbure fluoré.

6. Procédé selon l'une quelconque des revendications 1 à 3 dans lequel on modifie la distribution en fluor du composé hydrocarbure en isomérisant ledit composé hydrocarbure en présence de ladite composition solide, ledit composé hydrocarbure étant un composé hydrocarbure fluoré.

7. Procédé selon l'une quelconque des revendications 1 à 3 dans lequel on modifie la distribution en fluor du composé hydrocarbure en dismutant ledit composé hydrocarbure en phase gazeuse en présence de ladite composition solide, ledit composé hydrocarbure étant un composé hydrocarbure chlorofluoré.

8. Procédé selon l'une quelconque des revendications 1 à 3 dans lequel on diminue la teneur en fluor du composé hydrocarbure en faisant réagir ledit composé hydrocarbure avec du chlorure d'hydrogène en phase gazeuse en présence de ladite composition solide, ledit composé hydrocarbure étant un composé hydrocarbure halogéné contenant au moins un atome de fluor.

9. Procédé selon l'une quelconque des revendications 1 à 3 dans lequel on augmente la teneur en fluor d'un premier composé hydrocarbure en faisant réagir ledit premier composé hydrocarbure avec du fluorure d'hydrogène en phase gazeuse en présence d'une composition solide, le premier composé hydrocarbure étant un hydrocarbure saturé halogéné ou un hydrocarbure insaturé halogéné ou un hydrocarbure insaturé, et dans lequel on diminue la teneur en fluor d'un second composé hydrocarbure en déshydrofluorant ledit second composé hydrocarbure en présence de ladite composition solide, ledit second composé hydrocarbure étant un composé hydrocarbure fluoré.

10. Procédé selon l'une quelconque des revendications 1 à 3 dans lequel on augmente la teneur en fluor d'un premier composé hydrocarbure en faisant réagir ledit premier composé hydrocarbure avec du fluorure d'hydrogène en phase gazeuse en présence d'une composition solide, le premier composé hydrocarbure étant un hydrocarbure saturé halogéné ou un hydrocarbure insaturé halogéné ou un hydrocarbure insaturé, et dans lequel on diminue la teneur en chlore d'un second composé en déshydrochlorant ledit second composé hydrocarbure en présence de ladite composition solide, ledit second composé hydrocarbure étant un composé hydrocarbure fluoré.

11. Procédé selon l'une quelconque des revendications précédentes dans lequel le composant contenant de l'oxyfluorure ou du fluorure de chrome est de formule brute CrₓM(₁₋ₓ)OᵣFₛ, où r est supérieur ou égal à 0 et inférieur à 2,62, s est supérieur ou égal à [(0,16 r) + 0,52)]/0,92 sous réserve que s soit supérieur ou égal à 0,76 et inférieur à 6.

12. Procédé selon l'une quelconque des revendications précédentes dans lequel la composition solide contient un support choisi dans le groupe constitué de charbon actif, d'alumine, de fluorure de magnésium et de fluorure d'aluminium.

## Patentansprüche

1. Verfahren zur Modifizierung der Fluorverteilung in einer Kohlenwasserstoffverbindung der Formel (I) CX(Y)₂-CX(Y)ₘ-CHₘXY, wobei X und Y unabhängig für H, F oder Cl stehen und m = 0 oder 1, wobei mindestens eines von X oder Y C1 oder F ist, in Gegenwart eines Katalysators, wobei es sich bei dem Katalysator um eine feste Zusammensetzung handelt, die mindestens eine Komponente enthält, die Chromoxidfluorid oder -fluorid mit der Summenformel CrₓM(₁₋ₓ)OᵣFₛ enthält, wobei 2r+s größer als oder gleich 2,9 und kleiner als 6 ist, M Zn ist, x einen Wert von 0,9 bis 0,999 hat, s größer als 0 und kleiner als oder gleich 6 ist und r größer als oder gleich 0 und kleiner als 3 ist, **dadurch gekennzeichnet, dass** die Komponente, die Chromoxidfluorid oder -fluorid enthält, amorph ist und dass die feste Zusammensetzung eine spezifische Oberfläche zwischen 15 und 45 m²/g aufweist.

2. Verfahren nach einem der vorhergehenden Ansprüche, wobei die Komponente, die Chromoxidfluorid oder -fluorid enthält, die Summenformel CrₓM(₁₋ₓ)OᵣFₛ mit M = Zn aufweist; wobei x insbesondere einen Wert von 0,94 bis 0,99 hat.

3. Verfahren nach dem vorhergehenden Anspruch, wobei die Kohlenwasserstoffverbindung aus der Gruppe bestehend aus Tetrachlorpropen, Chlortrifluorpropen, Pentachlorpropan, Dichlortrifluorpropan, Trichlordifluorpropan, Tetrafluorchlorpropan, Tetrachlorfluorpropan, Dichlordifluorpropen, Trichlorfluorpropen, Pentafluorpropan und Mischungen davon ausgewählt wird; vorzugsweise die Kohlenwasserstoffverbindung aus der Gruppe bestehend aus 2-Chlor-3,3,3-trifluor-1-propen (HFCO-1233xf), 2,3-Dichlor-1,1,1-trifluorpropan (HCFC-243db), 1,1,1,2,3-Pentachlorpropan (HCC-240db), 1,1,2,2,3-Pentachlorpropan (HCC-240aa), 1,1,1,3,3-Pentachlorpropan (HCC-240fa), 1,1,2,3-Tetrachlor-1-propen (HCO-1230xa), 2,3,3,3-Tetrachlor-1-propen (HCO-1230xf), 1,1,3,3-Tetrachlor-1-propen (HCO-1230za), 1,3,3,3-Tetrachlor-1-propen (HCO-1230zd), 1,1,1,2,2-Pentafluorpropan (HFC-245cb) und 1-Chlor-3,3,3-trifluor-1-propen (HCFO-1233zd) ausgewählt wird.

4. Verfahren nach einem der vorhergehenden Ansprüche, wobei man den Fluorgehalt der Kohlenwasserstoffverbindung durch Umsetzen der Kohlenwasserstoffverbindung mit Fluorwasserstoff in der Gasphase in Gegenwart der festen Zusammensetzung erhöht, wobei es sich bei der Kohlenwasserstoffverbindung um einen gesättigten halogenierten Kohlenwasserstoff oder einen ungesättigten halogenierten Kohlenwasserstoff oder einen ungesättigten Kohlenwasserstoff handelt.

5. Verfahren nach einem der Ansprüche 1 bis 3, wobei man den Fluorgehalt der Kohlenwasserstoffverbindung durch Dehydrofluorierung der Kohlenwasserstoffverbindung in Gegenwart der festen Zusammensetzung verringert, wobei es sich bei der Kohlenwasserstoffverbindung um eine Fluorkohlenwasserstoffverbindung handelt.

6. Verfahren nach einem der Ansprüche 1 bis 3, wobei man die Fluorverteilung der Kohlenwasserstoffverbindung durch Isomerisieren der Kohlenwasserstoffverbindung in Gegenwart der festen Zusammensetzung modifiziert, wobei es sich bei der Kohlenwasserstoffverbindung um eine Fluorkohlenwasserstoffverbindung handelt.

7. Verfahren nach einem der Ansprüche 1 bis 3, wobei man die Fluorverteilung der Kohlenwasserstoffverbindung durch Dismutieren der Kohlenwasserstoffverbindung in der Gasphase in Gegenwart der festen Zusammensetzung modifiziert, wobei es sich bei der Kohlenwasserstoffverbindung um eine Chlorfluorkohlenwasserstoffverbindung handelt.

8. Verfahren nach einem der Ansprüche 1 bis 3, wobei man den Fluorgehalt der Kohlenwasserstoffverbindung durch Umsetzen der Kohlenwasserstoffverbindung mit Chlorwasserstoff in der Gasphase in Gegenwart der festen Zusammensetzung verringert, wobei es sich bei der Kohlenwasserstoffverbindung um eine halogenierte Kohlenwasserstoffverbindung mit mindestens einem Fluoratom handelt.

9. Verfahren nach einem der Ansprüche 1 bis 3, bei dem man den Fluorgehalt einer ersten Kohlenwasserstoffverbindung durch Umsetzen der ersten Kohlenwasserstoffverbindung mit Fluorwasserstoff in der Gasphase in Gegenwart einer festen Zusammensetzung erhöht, wobei es sich bei der ersten Kohlenwasserstoffverbindung um einen gesättigten halogenierten Kohlenwasserstoff oder einen ungesättigten halogenierten Kohlenwasserstoff oder einen ungesättigten Kohlenwasserstoff handelt, und bei dem man den Fluorgehalt einer zweiten Kohlenwasserstoffverbindung durch Dehydrofluorieren der zweiten Kohlenwasserstoffverbindung in Gegenwart der festen Zusammensetzung verringert, wobei es sich bei der Kohlenwasserstoffverbindung um eine Fluorkohlenwasserstoffverbindung handelt.

10. Verfahren nach einem der Ansprüche 1 bis 3, bei dem man den Fluorgehalt einer ersten Kohlenwasserstoffverbindung durch Umsetzen der ersten Kohlenwasserstoffverbindung mit Fluorwasserstoff in der Gasphase in Gegenwart einer festen Zusammensetzung erhöht, wobei es sich bei der ersten Kohlenwasserstoffverbindung um einen gesättigten halogenierten Kohlenwasserstoff oder einen ungesättigten halogenierten Kohlenwasserstoff oder einen ungesättigten Kohlenwasserstoff handelt, und bei dem man den Chlorgehalt einer zweiten Verbindung durch Dehydrochlorieren der zweiten Kohlenwasserstoffverbindung in Gegenwart der festen Zusammensetzung verringert, wobei es sich bei der zweiten Kohlenwasserstoffverbindung um eine Fluorkohlenwasserstoffverbindung handelt.

11. Verfahren nach einem der vorhergehenden Ansprüche, wobei die Komponente, die Chromoxidfluorid oder -fluorid enthält, die Summenformel CrₓM(₁₋ₓ)OᵣFₛ aufweist, wobei r größer als oder gleich 0 und kleiner als 2,62 ist, s größer als oder gleich [(0,16 r) + 0,52)]/0,92 ist, mit der Maßgabe, dass s größer als oder gleich 0,76 und kleiner als 6 ist.

12. Verfahren nach einem der vorhergehenden Ansprüche, wobei die feste Zusammensetzung einen Träger aus der Gruppe bestehend aus Aktivkohle, Aluminiumoxid, Magnesiumfluorid und Aluminiumfluorid enthält.

## Claims

1. Process for modifying the fluorine distribution in a hydrocarbon compound of formula (I) CX(Y)₂-CX(Y)ₘ-CHₘXY, where X and Y independently represent H, F or Cl and m = 0 or 1 with at least one from X or Y which is Cl or F; in the presence of a catalyst, the catalyst being a solid composition containing at least one component containing chromium oxyfluoride or fluoride of empirical formula CrₓM(₁₋ₓ)OᵣFₛ, where 2r + s is greater than or equal to 2.9 and less than 6, M is Zn, x has a value from 0.9 to 0.999, s is greater than 0 and less than or equal to 6 and r is greater than or equal to 0 and less than 3, **characterized in that** the said component containing chromium oxyfluoride or fluoride is amorphous and **in that** the solid composition has a specific surface between 15 and 45 m²/g.

2. Process according to any one of the preceding claims, in which the said component containing chromium oxyfluoride or fluoride is of empirical formula CrₓM(₁₋ₓ) O_{f}Fₛ, with M = Zn; in particular, x has a value from 0.94 to 0.99.

3. Process according to the preceding claim, in which the hydrocarbon compound is chosen from the group consisting of tetrachloropropene, chlorotrifluoropropene, pentachloropropane, dichlorotrifluoropropane, trichlorodifluoropropane, tetrafluorochloropropane, tetrachlorofluoropropane, dichlorodifluoropropene, trichlorofluoropropene, pentafluoropropane and their mixtures; preferably, the hydrocarbon compound is chosen from the group consisting of 2-chloro-3,3,3-trifluoro-1-propene (HFCO-1233xf), 2.3-dichloro-1.1.1-trifluoropropane (HCFC-243db), 1,1,1,2,3-pentachloropropane (HCC-240db), 1,1,2,2,3-pentachloropropane (HCC-240aa), 1,1,1,3,3-pentachloropropane (HCC-240fa), 1,1,2,3-tetrachloro-1-propene (HCO-1230xa), 2,3,3,3-tetrachloro-1-propene (HCO-1230xf), 1,1,3,3-tetrachloro-1-propene (HCO-1230za), 1,3,3,3-tetrachloro-1-propene (HCO-1230zd), 1,1,1,2,2-pentafluoropropane (HFC-245cb) and 1-chloro-3,3,3-trifluoro-1-propene (HCFO-1233zd) .

4. Process according to any one of the preceding claims, in which the fluorine content of the hydrocarbon compound is increased by reacting the said hydrocarbon compound with hydrogen fluoride in the gas phase in the presence of the said solid composition, the hydrocarbon compound being a saturated halogenated hydrocarbon or an unsaturated halogenated hydrocarbon or an unsaturated hydrocarbon.

5. Process according to any one of Claims 1 to 3, in which the fluorine content of the hydrocarbon compound is reduced by dehydrofluorination of the said hydrocarbon compound in the presence of the said solid composition, the said hydrocarbon compound being a fluorinated hydrocarbon compound.

6. Process according to any one of Claims 1 to 3, in which the fluorine distribution of the hydrocarbon compound is modified by isomerizing the said hydrocarbon compound in the presence of the said solid composition, the said hydrocarbon compound being a fluorinated hydrocarbon compound.

7. Process according to any one of Claims 1 to 3, in which the fluorine distribution of the hydrocarbon compound is modified by disproportionating the said hydrocarbon compound in the gas phase in the presence of the said solid composition, the said hydrocarbon compound being a chlorofluorinated hydrocarbon compound.

8. Process according to any one of Claims 1 to 3, in which the fluorine content of the hydrocarbon compound is decreased by reacting the said hydrocarbon compound with hydrogen chloride in the gas phase in the presence of the said solid composition, the said hydrocarbon compound being a halogenated hydrocarbon compound containing at least one fluorine atom.

9. Process according to any one of Claims 1 to 3, in which the fluorine content of a first hydrocarbon compound is increased by reacting the said first hydrocarbon compound with hydrogen fluoride in the gas phase in the presence of a solid composition, the first hydrocarbon compound being a saturated halogenated hydrocarbon or an unsaturated halogenated hydrocarbon or an unsaturated hydrocarbon, and in which the fluorine content of a second hydrocarbon compound is reduced by dehydrofluorinating the said second hydrocarbon compound in the presence of the said solid composition, the said second hydrocarbon compound being a fluorinated hydrocarbon compound.

10. Process according to any one of Claims 1 to 3, in which the fluorine content of a first hydrocarbon compound is increased by reacting the said first hydrocarbon compound with hydrogen fluoride in the gas phase in the presence of a solid composition, the first hydrocarbon compound being a saturated halogenated hydrocarbon or an unsaturated halogenated hydrocarbon or an unsaturated hydrocarbon, and in which the chlorine content of a second compound is reduced by dehydrochlorinating the said second hydrocarbon compound in the presence of the said solid composition, the said second hydrocarbon compound being a fluorinated hydrocarbon compound.

11. Process according to any one of the preceding claims, in which the component containing chromium oxyfluoride or fluoride is of empirical formula CrₓM(₁₋ₓ)OᵣFₛ, where r is greater than or equal to 0 and less than 2.62, s is greater than or equal to [(0.16 r) + 0.52)]/0.92, on condition that s is greater than or equal to 0.76 and less than 6.

12. Process according to any one of the preceding claims, in which the solid composition contains a support chosen from the group consisting of activated carbon, alumina, magnesium fluoride and aluminium fluoride.
